(19) Europäisches Patentamt
European Patent Office
Office européen des brevets

(11) EP 1 052 261 A1

(12) # EUROPEAN PATENT APPLICATION
published in accordance with Art. 158(3) EPC

(43) Date of publication:
**15.11.2000 Bulletin 2000/46**

(21) Application number: **99902822.8**

(22) Date of filing: **03.02.1999**

(51) Int. Cl.[7]: **C07D 405/14**, C07D 407/06, C07D 413/14, C07D 417/14, C07D 491/052, C07D 491/16, C08F 2/50, C08K 5/18, G03F 7/031, H01S 3/16

(86) International application number:
**PCT/JP99/00459**

(87) International publication number:
**WO 99/40086 (12.08.1999 Gazette 1999/32)**

(84) Designated Contracting States:
**DE FR GB NL**

(30) Priority: **03.02.1998 JP 6029798**
**10.09.1998 JP 29752198**

(71) Applicant:
**Kabushiki Kaisha Hayashibara Seibutsu Kagaku Kenkyujo**
**Okayama-shi, Okayama 700-0907 (JP)**

(72) Inventors:
- **MITEKURA, Hirofumi**
  **Okayama-shi, Okayama 701-1145 (JP)**
- **DAN-OH, Yasufumi**
  **Okayama-shi, Okayama 702-8055 (JP)**
- **OOGA, Yasuyo**
  **Kurashiki-shi, Okayama 711-0927 (JP)**
- **MORI, Naoko**
  **Okayama-shi, Okayama 702-8055 (JP)**
- **SUGA, Sadaharu**
  **Tamano-shi, Okayama 706-0011 (JP)**
- **TANIGUCHI, Yoshio**
  **Ueda-shi, Nagano 386-0012 (JP)**
- **KOYAMA, Toshiki**
  **Ueda-shi, Nagano 386-0003 (JP)**
- **ADACHI, Chihaya**
  **Ueda-shi, Nagano 386-0015 (JP)**
- **SAITOU, Tomoyasu**
  **Urawa-shi, Saitama 338-0832 (JP)**
- **SATSUKI, Makoto**
  **Tsukubo-gun, Okayama 719-1100 (JP)**

(74) Representative:
**Jenkins, Peter David et al**
**PAGE WHITE & FARRER**
**54 Doughty Street**
**London WC1N 2LS (GB)**

(54) **PYRAN DERIVATIVES AND USE THEREOF**

(57) Pyran derivatives, which are obtainable through a step of reacting a compound having 3-formylcoumarin skeleton with either dehydroacetic acid or a compound having 4-cyanomethylene-2,6-dimethyl-4H-pyran skeleton, have a distinctive sensitivity to visible light and a distinctive luminous ability, and thus they have various applicabilities in photopolymerization, electroluminescence, and dye lasers.

EP 1 052 261 A1

## Description

TECHNICAL FIELD

The present invention was made based on the discovery of novel pyran derivatives, and more particularly, it relates to novel pyran derivatives, which have distinctive sensitivity to visible light and distinctive luminous ability, and to their uses and processes.

BACKGROUND ART

After coming into this information age, photochemical polymerization has been frequently used in a variety of fields, and, beyond the field of synthetic resins, it has now been extensively used in other fields of information recordings and electric equipments such as paints, plates for printings, printing circuits, integrated circuits, etc. Photochemical polymerization is a technique for polymerizing polymerizable compounds by irradiating light, and is roughly classified into photopolymerization which initiates to polymerize polymerizable compounds by directly irradiating light to activate them, and photosensitizing polymerization which polymerizes polymerizable compounds by irradiating light in the presence of photosensitizers to form growth active-cores of photosensitizers. Both of these photochemical polymerizations have features that their polymerization initiation and suspension can be controlled by flashing excitation light, and the polymerization rate and degree can be easily controlled by selecting the excitation strength and wavelength. Photochemical polymerization can also be proceeded even at relatively-low temperatures because of its relatively-low polymerization initiation energy.
At present, owing to the above advantageous features of photopolymerization, photopolymerizable compositions, which can be polymerized by irradiating visible light such as argon lasers, helium/neon lasers, and second harmonic of YAG lasers, have rapidly being increased as the new development of information recordings such as plates for printings and holographies. However, since conventional polymerizable compounds and polymerization initiators, which are incorporated in photopolymerizable compositions, absorb ultraviolet rays only, they inevitably require photosensitizers as an essential technical factor when polymerized by visible light. Accordingly, in the photopolymerizable compositions used in information recordings and electric equipments, polymerizable compounds should be used in combination with photosensitizers, polymerization initiators, binding resins, etc., which are all selected from among various materials depending on their use. In general, methods, which comprise the steps of successively selecting materials other than photosensitizers and selecting the desired photosensitizers capable of sensitizing the selected polymerizable compounds and/or polymerization initiators in a manner of trial and error, can be selected.
The properties desirable for photosensitizers are as follows: A relatively-large molecular extinction coefficient in visible region, ability of sensitizing polymerizable compounds and polymerization initiators, higher sensitization efficiency, superior solubility in solvents and compatibility with other components, and stability. Representative examples of such photosensitizers are, for example, merocyanine dyes as disclosed in Japanese Patent Kokai No. 151,024/79, cyanine dyes as disclosed in Japanese Patent Kokai No. 29,803/83, stilbene derivatives as disclosed in Japanese Patent Kokai No. 56,403/84, coumarin derivatives as disclosed in Japanese Patent Kokai No. 23,901/88, pyran derivatives as disclosed in Japanese Patent Kokai No. 329,654/94, and methylene blue derivatives as disclosed in Japanese Patent Kokai No. 33,104/89. All of these photosensitizers, however, have both merits and demerits, and there has not yet been found any photosensitizer, which can constantly exert the above properties when used in photopolymerizable compositions comprising various materials.
Organic compounds sensitive to light, particularly, luminescent organic compounds are useful in the field of dye lasers and electroluminescence.
In the field of dye lasers, as disclosed, for example, by Kaoru IGARASHI in "*Shikizai-Kyokai-shi*", Vol. 70, No. 2, pp. 102-111 (1997), compounds which emit a light in a visible region have been eagerly screened since the report of dye laser's lasing in 1960's. As the progress of information recording technology, compounds which emit a light in a longer wavelength region, and more particularly, those which emit a light in a visible region have been demanded more and more.
In the field of information displays, electroluminescent devices are highlighted as displaying devices for the forthcoming generation. Today, cathode-ray tubes are predominantly used in a relatively-large size of information displaying means such as computer termini and TV receivers. The cathode-ray tubes, however, are relatively large in mass and weight and are relatively high in operation voltage, and this spoils their applicability to family-use equipments and smaller size of portable equipments where the transportability is highly valued. More required are information displaying means which have a thinner and lighter plain form and operate at a lower operation voltage and wattage. Because of the advantageous features of a relatively-low operation voltage and wattage, liquid crystal devices are now widely used in various fields. However, as a demerit, since the information displaying means equipped with the liquid crystal devices change in contrast depending on their view angles, they can display information clearly only when viewed within a specific view angle and generally require blacklight so that they could not effectively reduce their wattage as much as they are expected. A device, which is now focused as a displaying device in the forthcoming generation and which overcomes the above drawbacks, is an electroluminescent device or an organic EL device.

Such an organic EL device is a luminescent device which has a thin layer, containing a luminescent compound, inserted between a cathode and an anode, and which utilizes luminescence such as fluorescence or phosphorescence emitted by the luminescent compound when a dc voltage is first supplied between the cathode and the anode to supply positive holes and electrons to the thin layer and to couple again the positive holes and the electrons together to make the luminescent compound into an excited state, and then the excited compound returns to the ground state. As a characteristic feature, organic EL device can be appropriately changed in luminous color tint by selecting appropriate luminescent compounds and altering dye agents as dopants used in combination with the luminescent compounds. Depending on the combination of luminescent compounds and dye agents, the brightness and the life expectancy of luminescence can possibly be improved by a large margin. It is said that organic EL device is a theoretically-excellent luminescent device because of the following advantageous features: Since organic EL device autonomously emits light, information displaying means with such organic EL device are free of view angles and low in energy consumption because they need no back light.

Although, in organic EL device which emits light in a green color region, the improvement of luminescent efficiency by incorporating dye agents was reported, there has not yet been found any dye agent which effectively allows organic EL device to emit light in a red color region and is still be left far from emitting a complete red luminescence, poor in life expectancy, and insufficient in both durability and reliability. For example, the organic EL devices, as disclosed in Japanese Patent Kokai No. 6,042/98 and United States Patent No. 4,769,292, are insufficient in brightness and incomplete in red color emission, and hence they would still be problematic in realizing a full color emission.

To supply organic EL devices at a lower cost, it is essential to find luminescent compounds which do not intrinsically need doping by dye agents, let alone simplifying the systematic structure of the organic EL devices and facilitating the step of vapor deposition in their processings. Many proposals have been made on luminescent compounds used in organic EL devices, but there found no compound which fulfills the above requirements.

## DISCLOSURE OF INVENTION

In view of the foregoing, the objects of the present invention are to provide novel organic compounds, which have distinct sensitivity to visible light, significant luminous ability, and usefulness in photochemical polymerization, dye lasers, and organic EL devices; and to uses thereof as photosensitizers, organic EL devices, and dye lasers.

To solve the above objects, the present inventors eagerly screened compounds and researched to find that a series of pyran derivatives, which are obtainable through a step of reacting a compound having 3-formylcoumarin skeleton with a compound having either dehydroacetic acid or 4-cyanomethylene-2,6-dimethyl-4H-pyran skeleton, have distinct sensitivity to visible light and extreme usefulness in photochemical polymerization. They also found that the pyran derivatives have distinct luminous ability, impart actually-usable durability to the organic EL devices when used in organic EL devices, allow the devices to emit highly-bright complete or substantially complete red color, provide excellent displaying equipments by using the devices, and provide excellent dye laser oscillating apparatuses because they are extremely useful in dye lasers as laser active substances for oscillating visible light. The present invention was made based on the creation of novel pyran derivatives and the findings of their industrially-useful features.

## BRIEF DESCRIPTION OF DRAWING

FIG. 1 is a brief figure of the organic EL device of the present invention. In the figure, 1, 2, 3, 4, 5 and 6 represent a substrate, anode, hole transportation layer, luminous layer, electron transportation layer, and cathode, respectively.

## BEST MODE FOR CARRYING OUT THE INVENTION

The present invention solves the above objects by providing the following pyran derivatives represented by Formulae 1 to 3:

Formula 1:

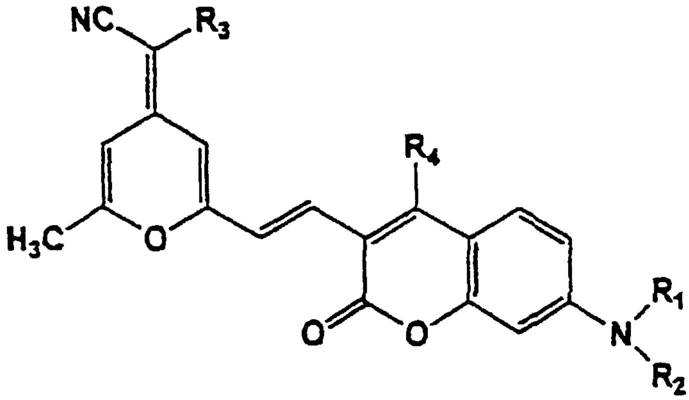

Formula 2:

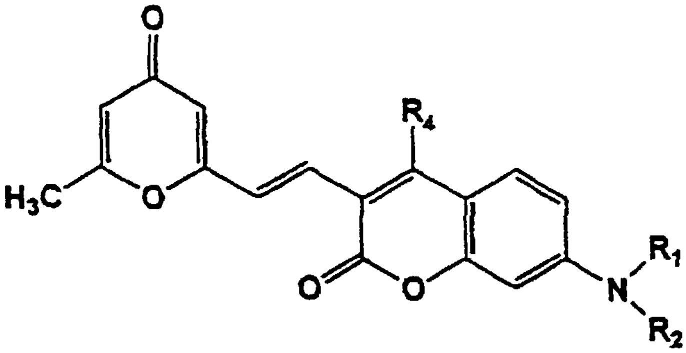

Formula 3:

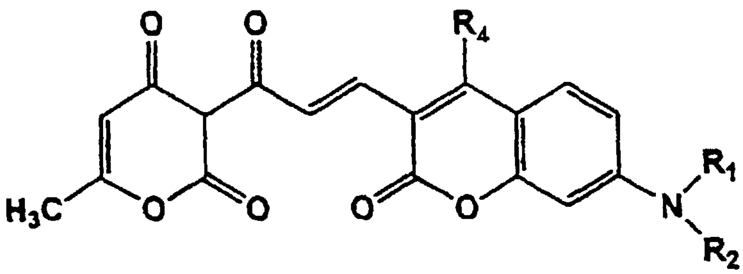

In Formulae 1 to 3, $R_1$ and $R_2$ independently denote hydrogen or a straight- or branched-chain of alkyl, alkenyl or aryl group, where the alkyl and aryl groups may contain a substituent. Depending on use, when $R_1$ and $R_2$ are alkyl groups, the chain length is usually selected from those up to 20 carbon atoms, and preferably selected from those of 1 to 18 carbon atoms. Respective examples of the straight- or branched-chain of alkyl group are methyl, ethyl, propyl, n-butyl, isobutyl, sec-butyl, pentyl, hexyl, heptyl, octyl, 2-ethyl-hexyl, 5-ethyl-hexyl, nonyl, decyl, dodecyl, and octadecyl groups. Examples of the alkenyl group are vinyl, propenyl, and butyl groups; the aryl group are phenyl and naphthyl groups. Substituents for the alkyl and aryl groups are, for example, alkoxy, carboxy, alkylcarbonyloxy, alkoxycarbonyl, sulfoxy, alkylsulfonyl, hydroxy, aryl, cycloalkyl, cyano, aminosulfonyl, 2-butoxyethyl, 2-(2-ethoxy)ethoxyethyl, 2-cyanoethyl, 6-bromohexyl, 2-carboxyethyl, 3-sulfoxypropyl, 4-sulfoxybutyl, 2-hydroxyethyl, phenylmethyl, 4-butoxyphenylmethyl, and 4-butylphenylmethyl groups. $R_1$ and/or $R_2$ may form five- or six-membered cyclic structures such as morpholinyl, piperidinyl, and pyrrolidinyl groups in cooperation with the nitrogen atoms bonded thereunto; or may form

five- or six-membered cyclic structures in cooperation with the benzene ring bonded to the nitrogen atoms.

In Formula 1, $R_3$ denotes cyano, carboxyl, carboxylic ester, or amido group; or a five- or six-membered heterocycle which may form a fused ring. Examples of such a heterocycle are benzothiazolyl, bentoxazolyl, benzoimidazolyl, naphtothiazolyl, naphtoxazolyl, napthoimidazolyl, thiazolyl, oxazolyl, imidazolyl, and triazolyl groups. These heterocycles, which may contain such a fused ring, may have one or more substituents such as alkyl, alkoxy, aryl, halogen-substituted alkyl, organic acid ester, halogeno, nitro, cyano, trifluoroalkyl, carboxylic acid and/or sulfonic acid groups.

Furthermore, in Formulae 1 to 3, $R_4$ denotes hydrogen atom; a halogen atom such as fluorine, chlorine, bromine, or iodine atom; cyano group; alkyl or carbonyl group having up to six carbon atoms; carbonyl group such as alkoxycarbonyl, acyl, or amido group; or a substituent derivable from the carbonyl group; where the alkyl group may have a straight-, cyclic-, or branched-chain structure or have a substituent such as halogen including fluorine.

Preferable examples of the pyran derivatives represented by Formulae 1 to 3 are the compounds represented by the following Formulae 4 to 59. Among these pyran derivatives, those, which have a distinctive luminous ability, for example, the compounds represented by Formulae 5, 35 and 49, are extremely useful in dye lasers and organic EL devices; and those, which have a distinctive sensitivity to visible light, for example, the compounds represented by Formulae 5, 6, 8, 10, 16, 17, 49, 50, 52 and 54 can be extremely-advantageously used as photosensitizers to polymerize polymerizable compounds photochemically.

Formula 4:

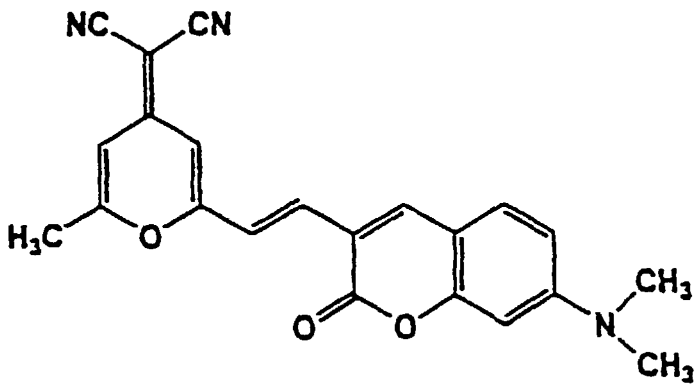

Formula 5:

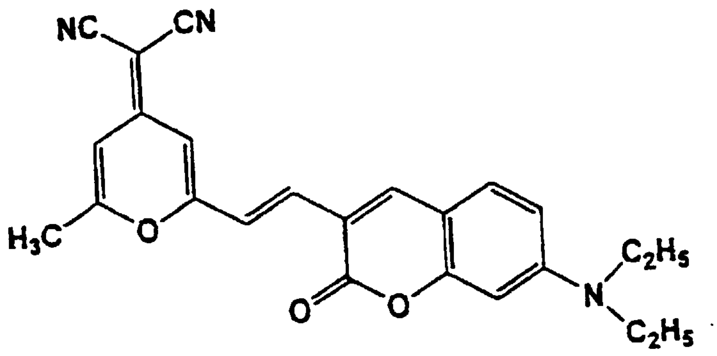

Formula 6:

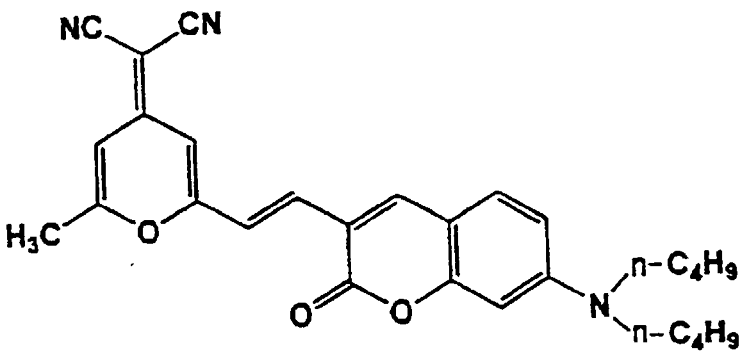

Formula 7:

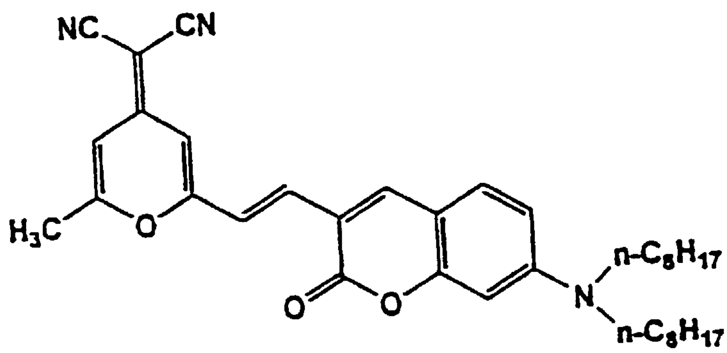

Formula 8:

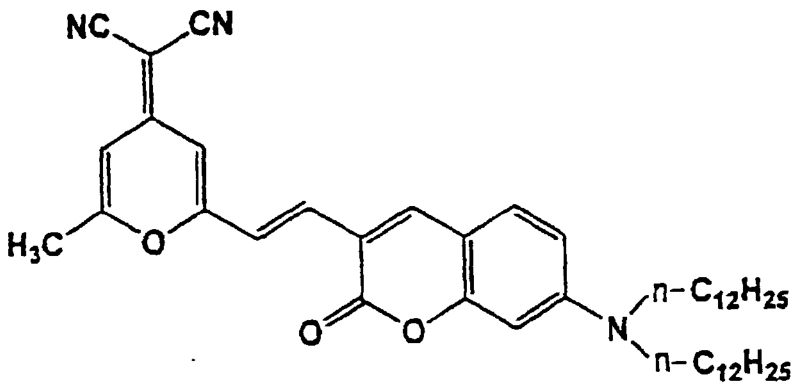

Formula 9:

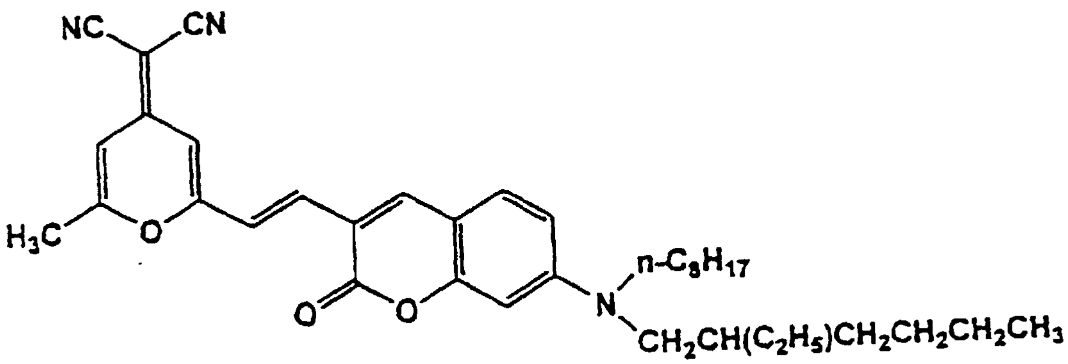

Formula 10:

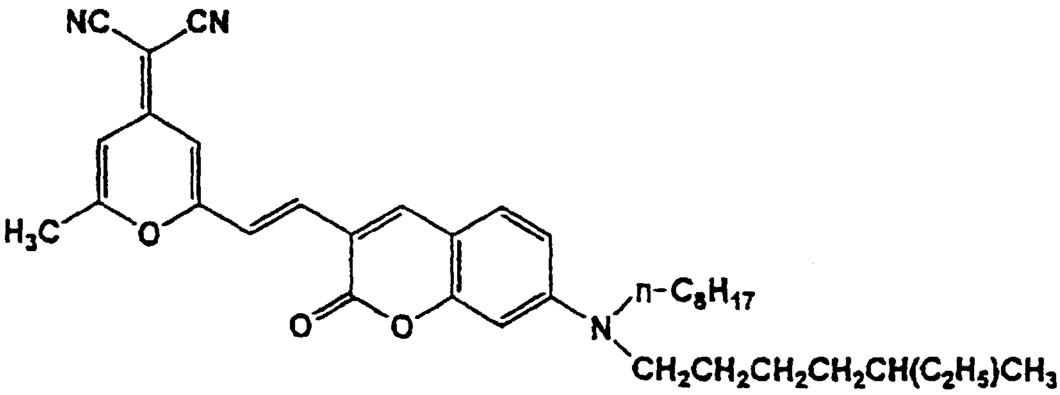

Formula 11:

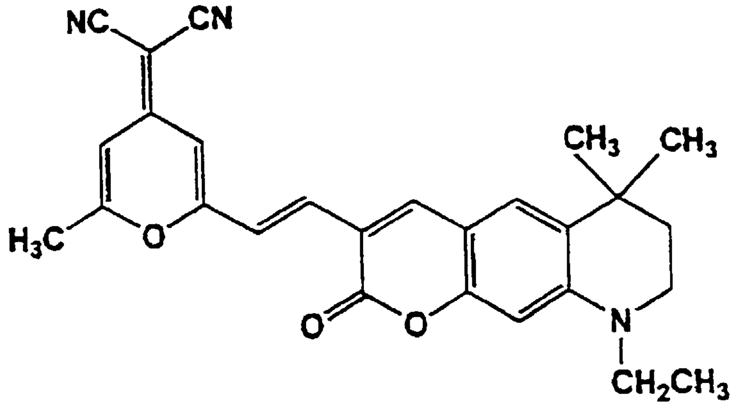

Formula 12:

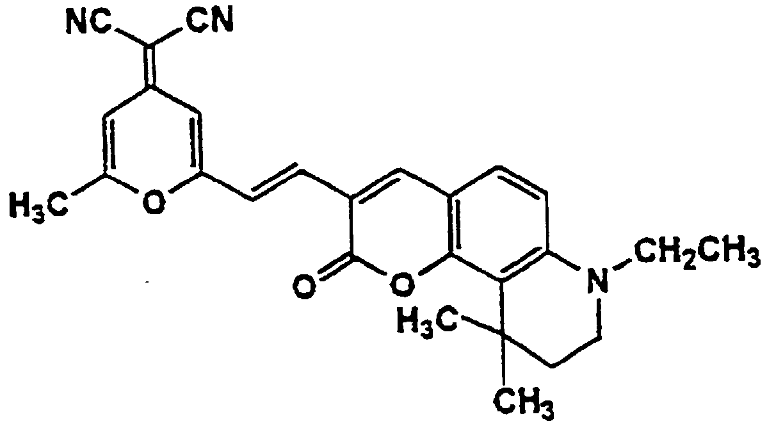

Formula 13:

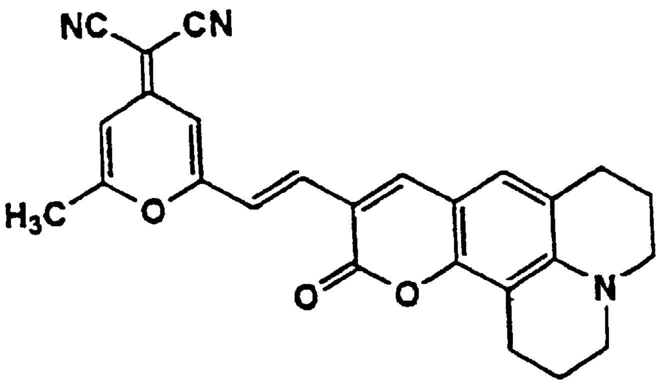

Formula 14:

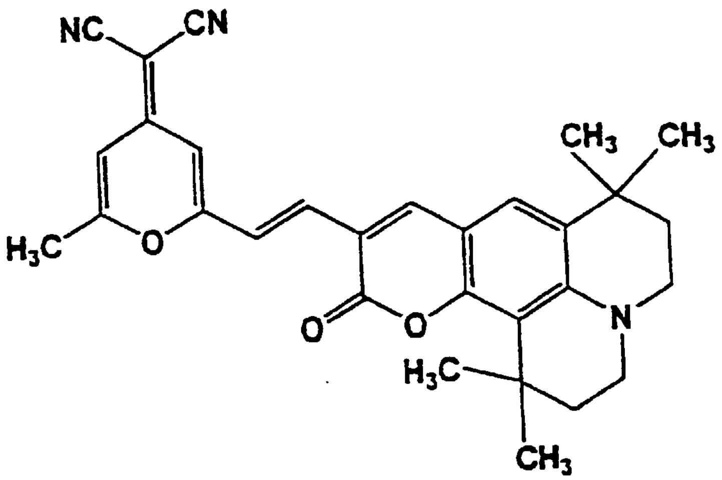

Formula 15:

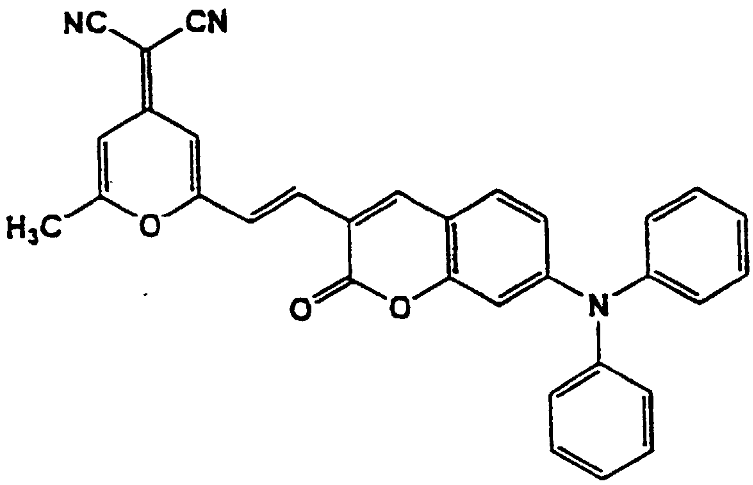

Formula 16:

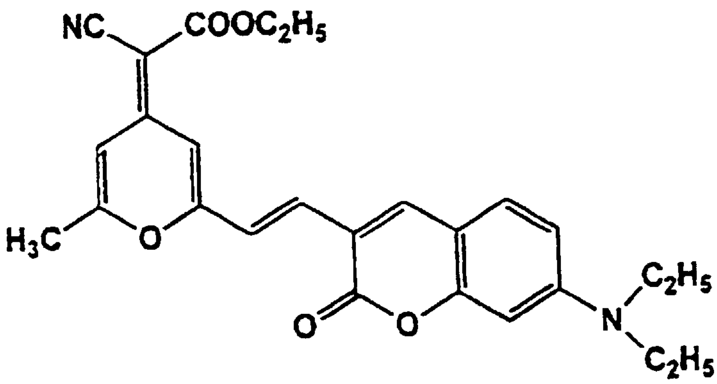

Formula 17:

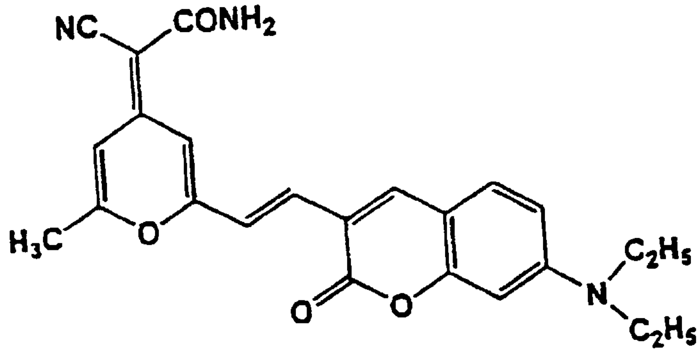

Formula 18:

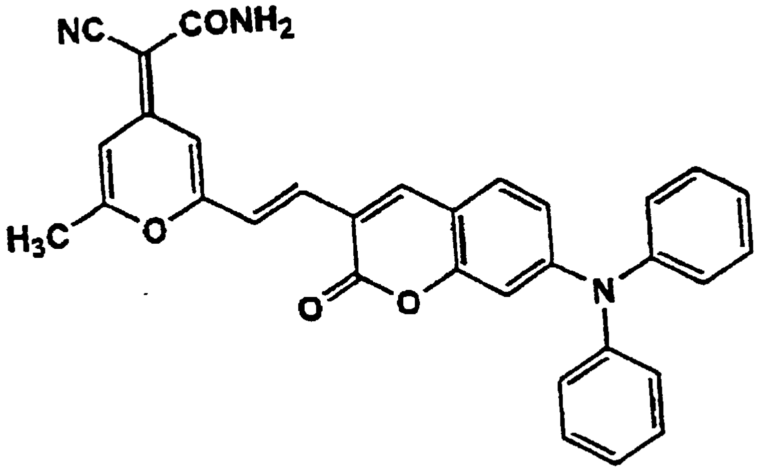

Formula 19:

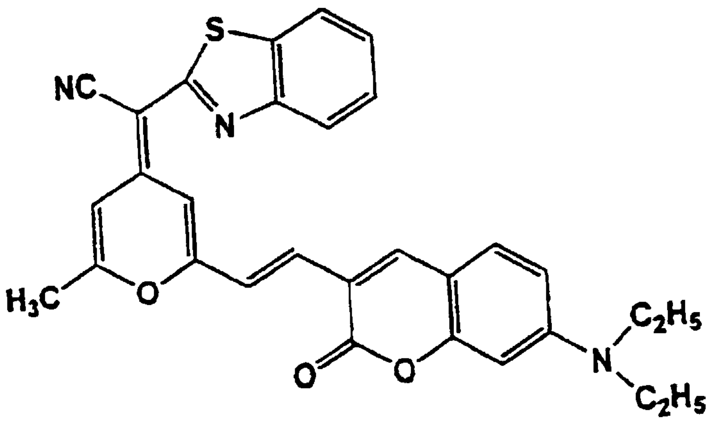

Formula 20:

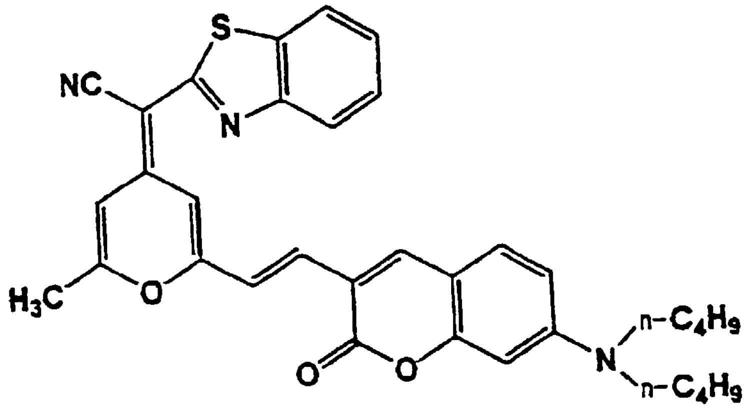

Formula 21:

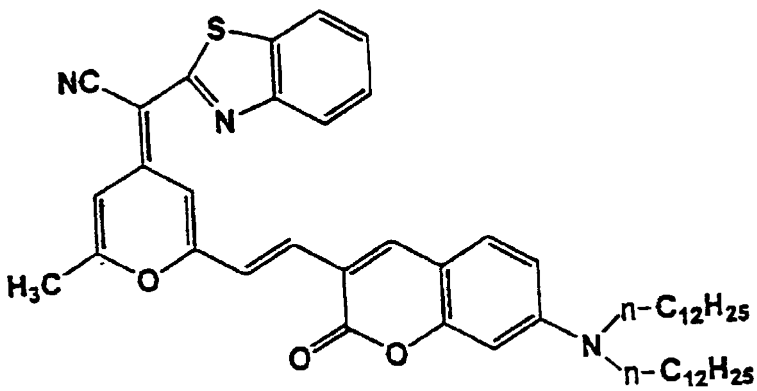

Formula 22:

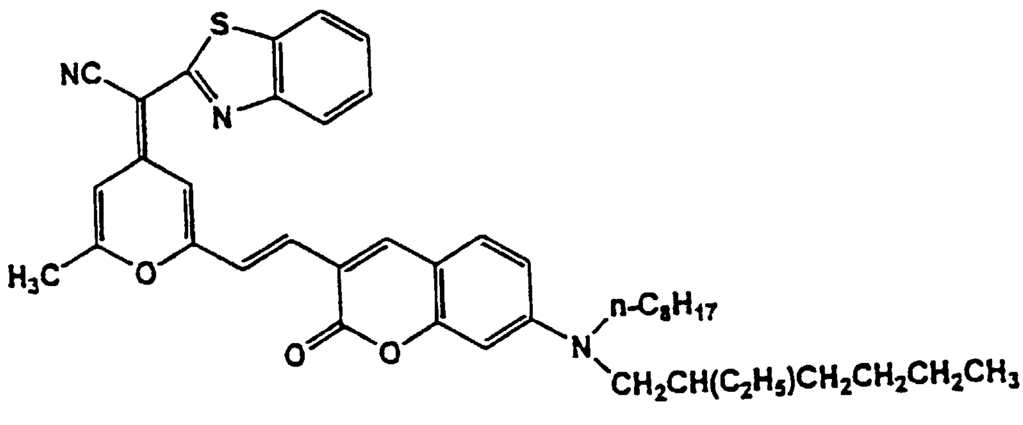

Formula 23:

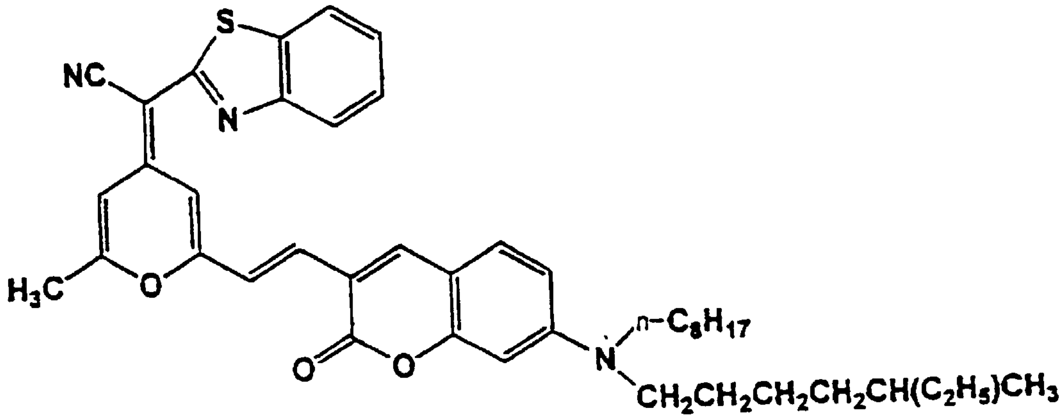

Formula 24:

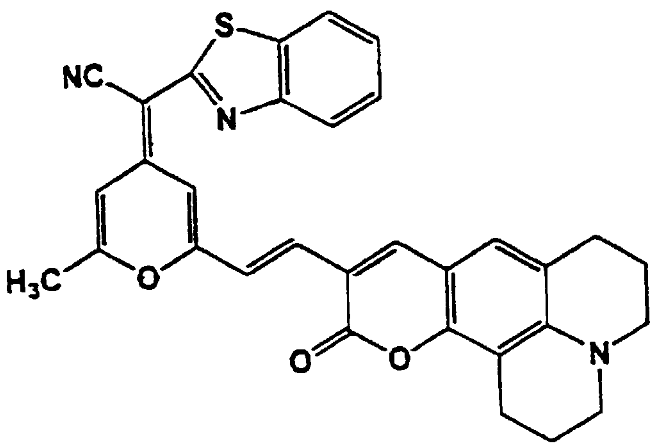

Formula 25:

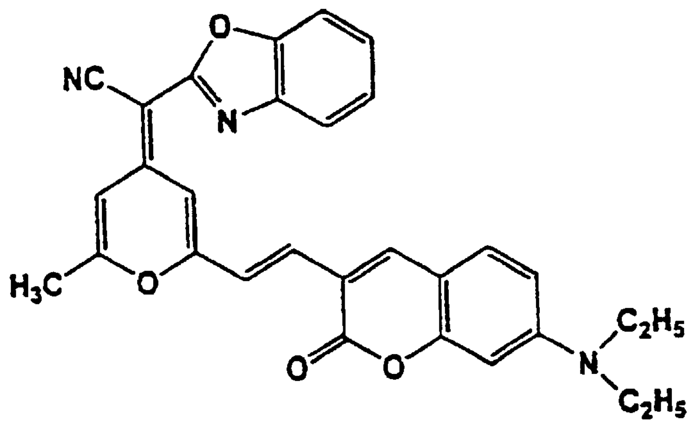

Formula 26

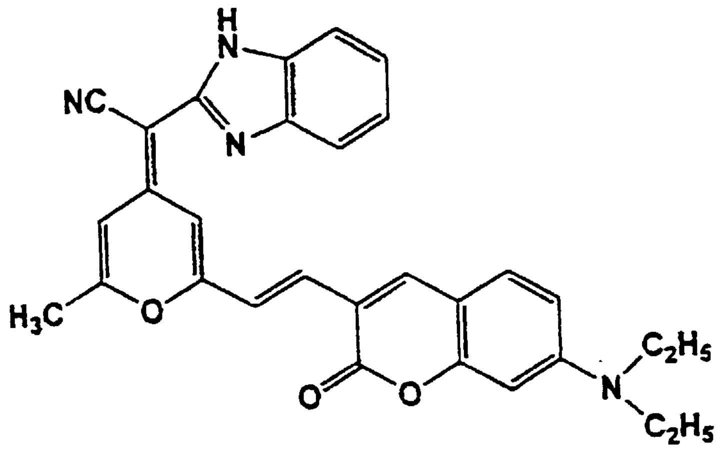

Formula 27:

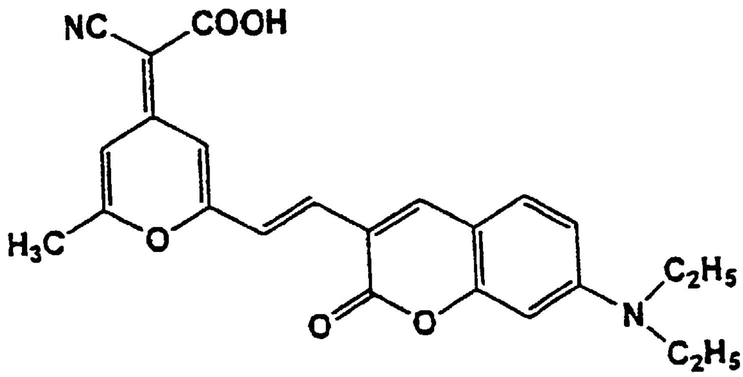

Formula 28:

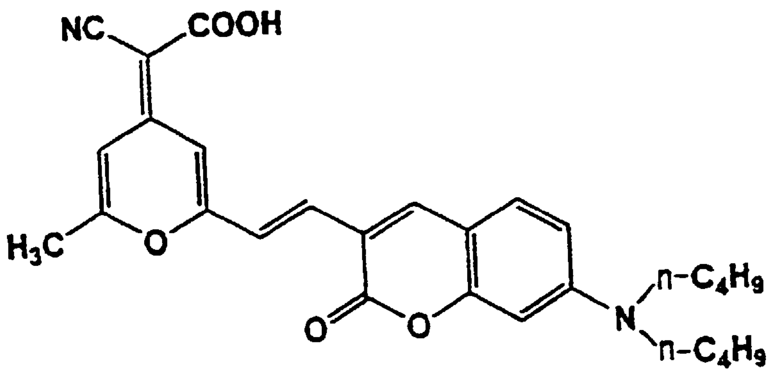

Formula 29:

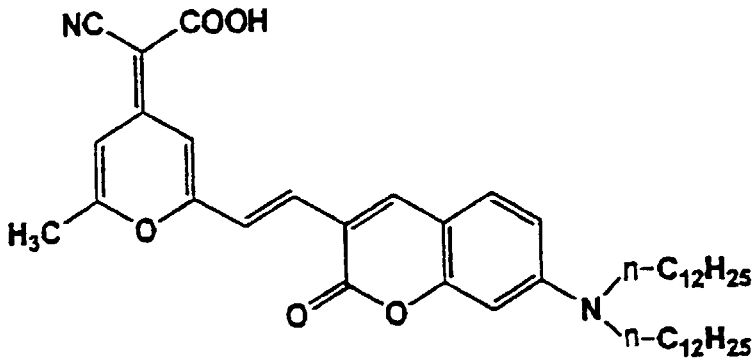

Formula 30:

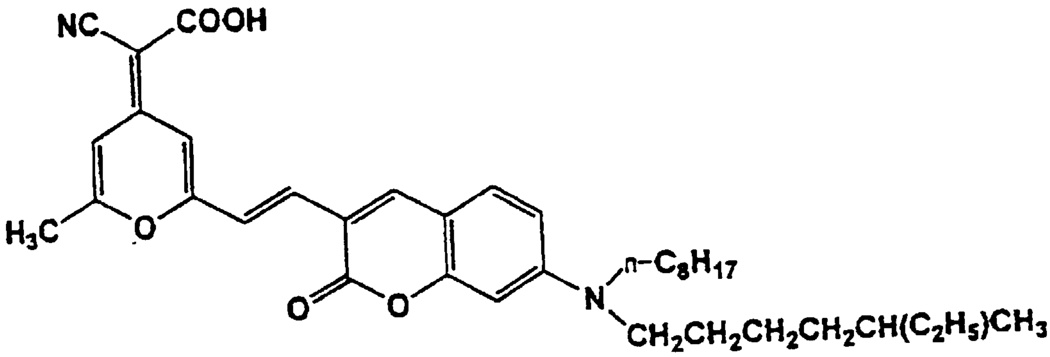

Formula 31:

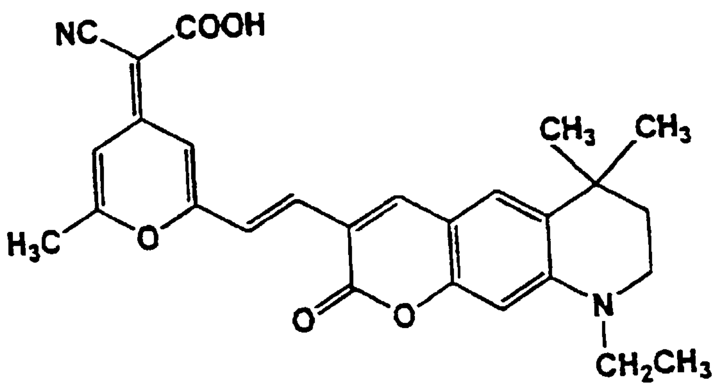

Formula 32:

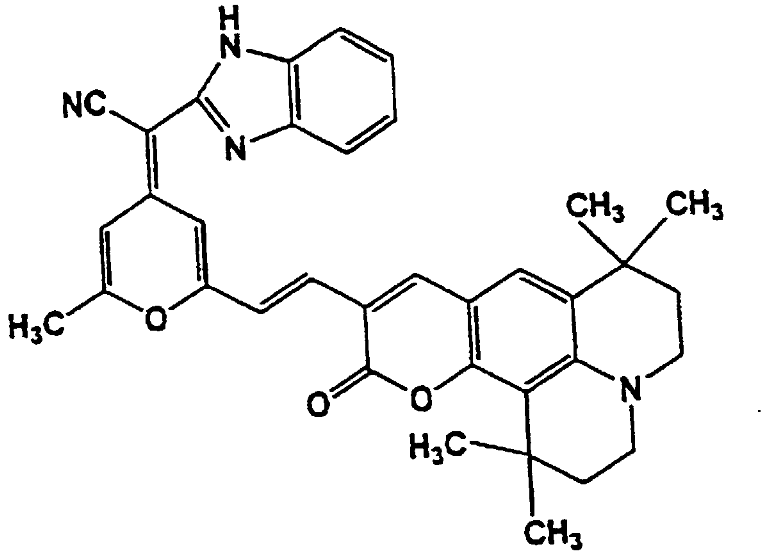

Formula 33:

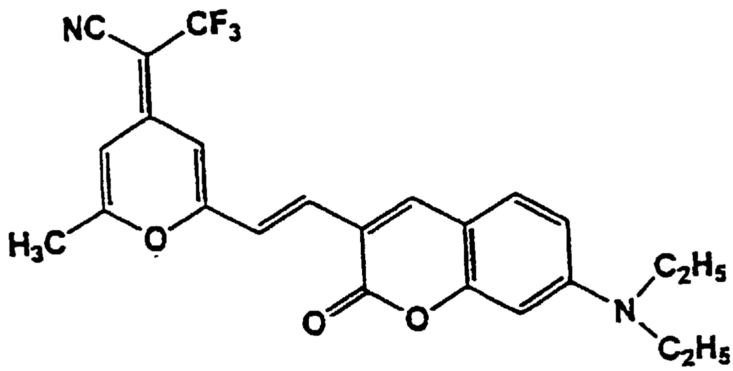

Formula 34:

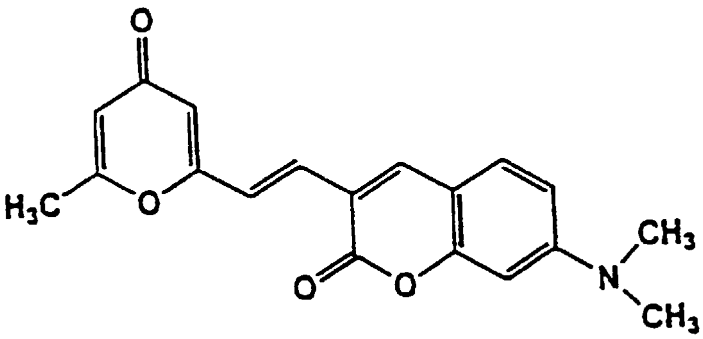

Formula 35:

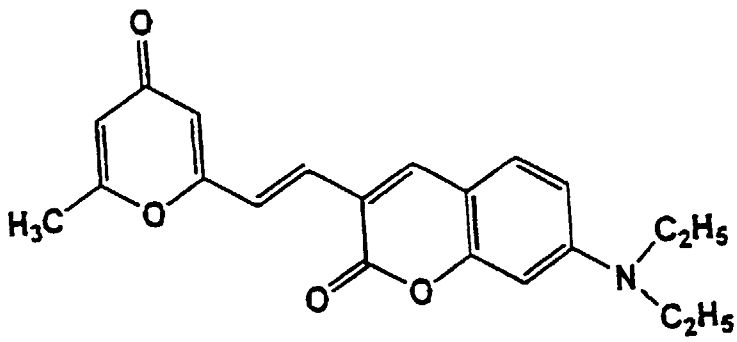

Formula 36:

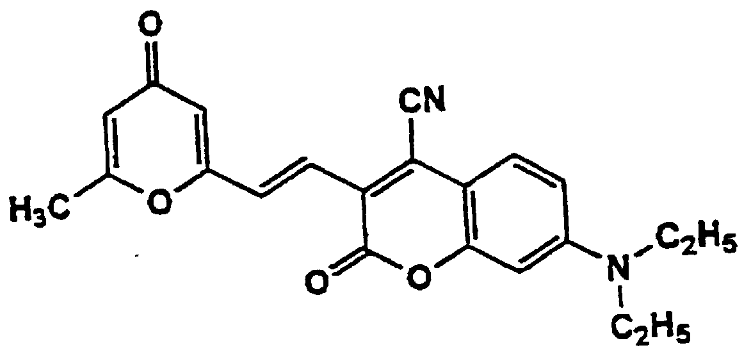

Formula 37:

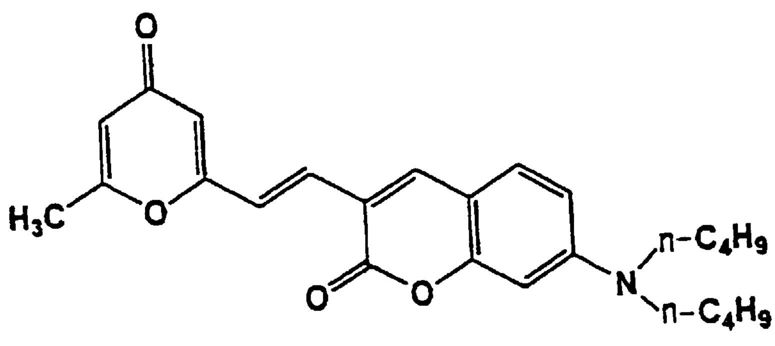

Formula 38:

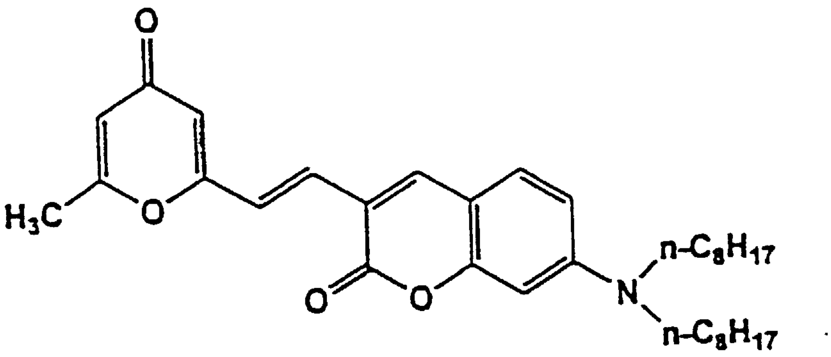

Formula 39:

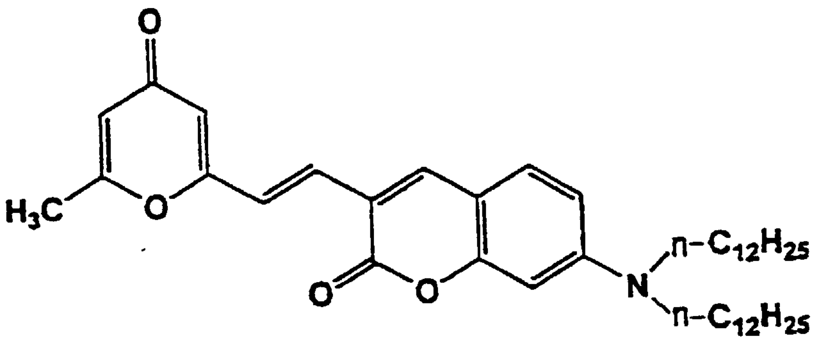

Formula 40:

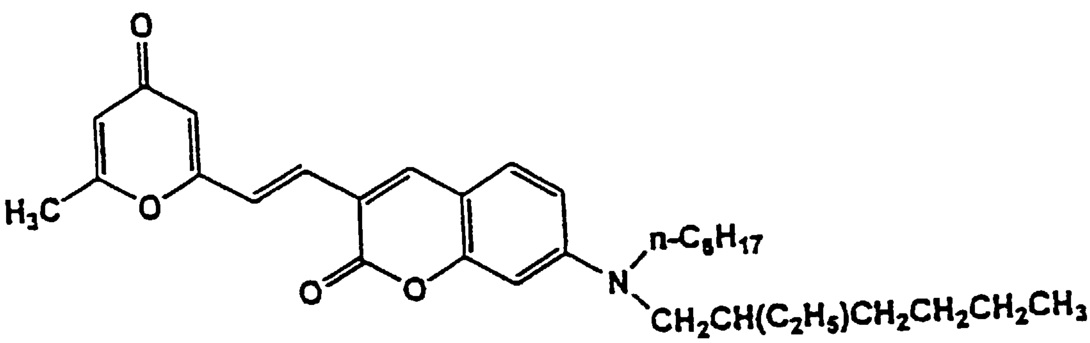

Formula 41:

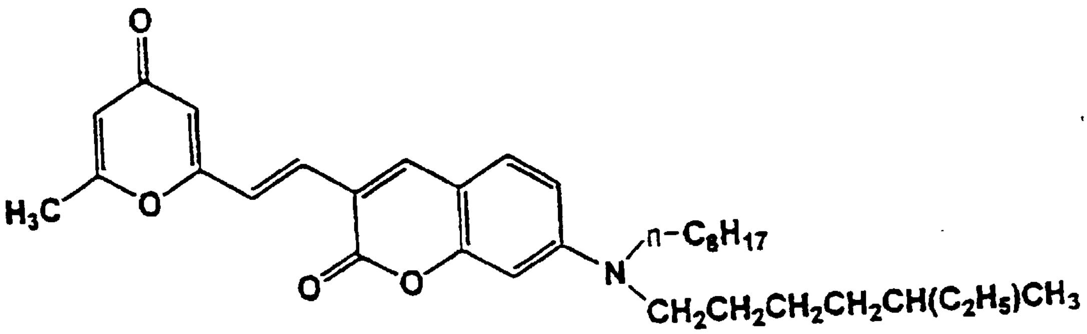

Formula 42:

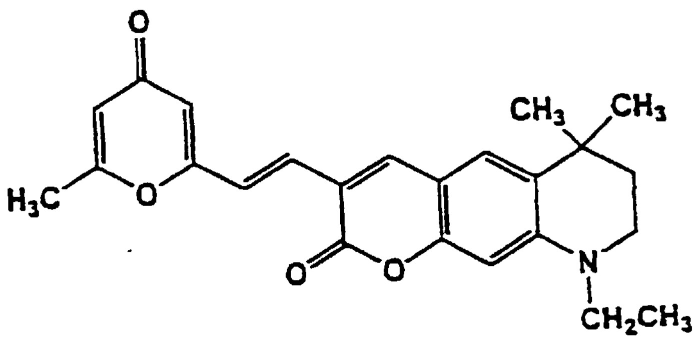

Formula 43:

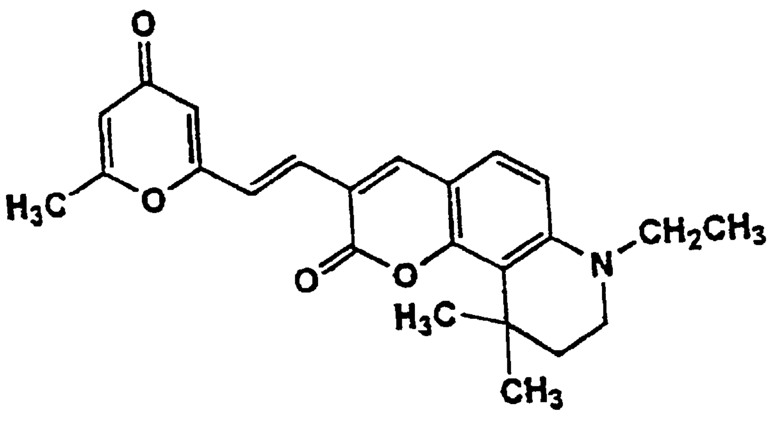

Formula 44:

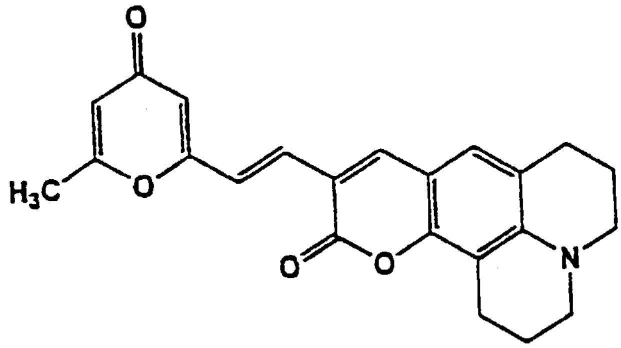

Formula 45:

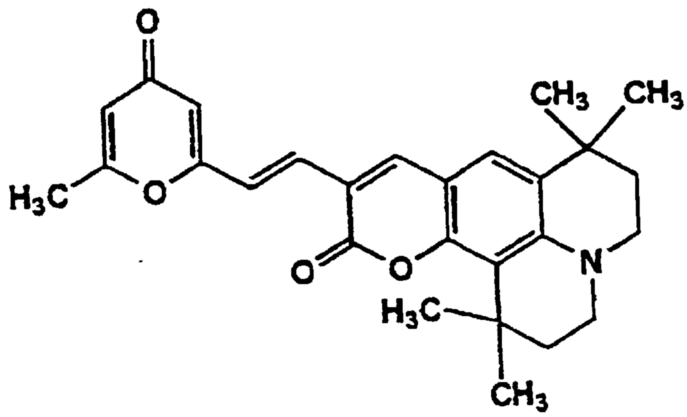

Formula 46:

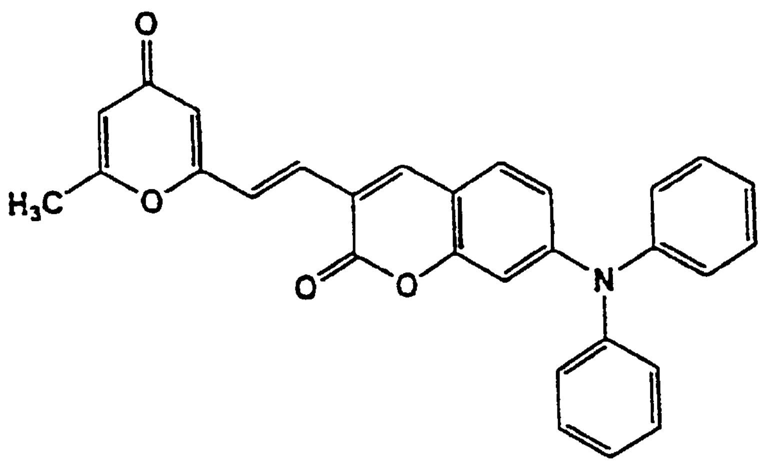

Formula 47:

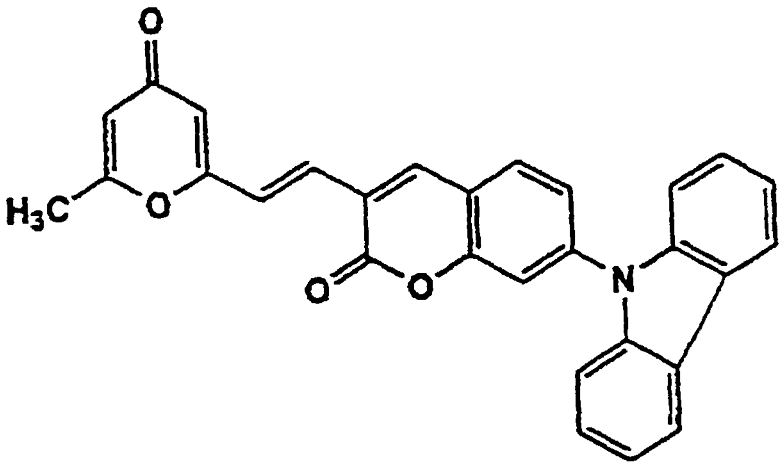

Formula 48:

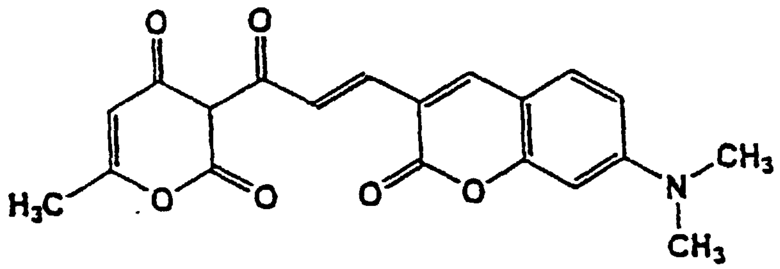

Formula 49:

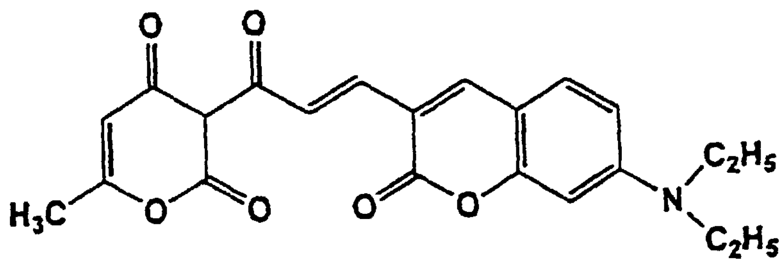

Formula 50:

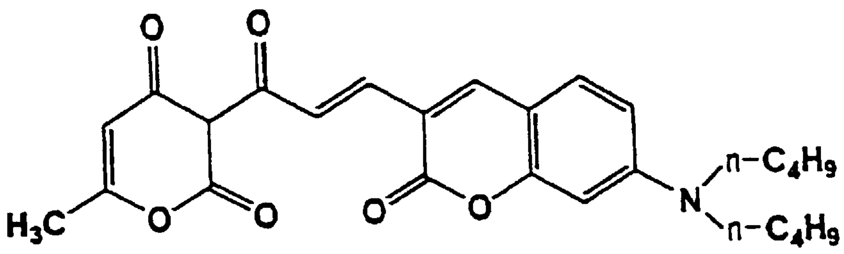

Formula 51:

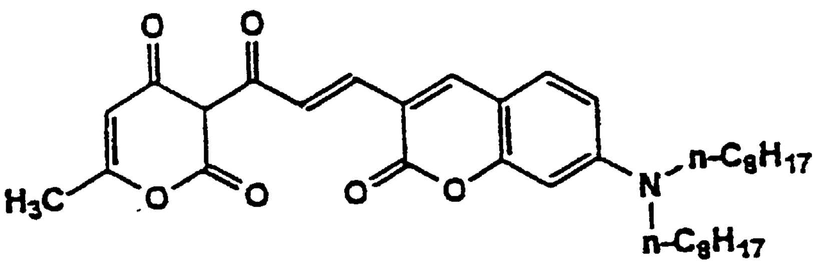

Formula 52:

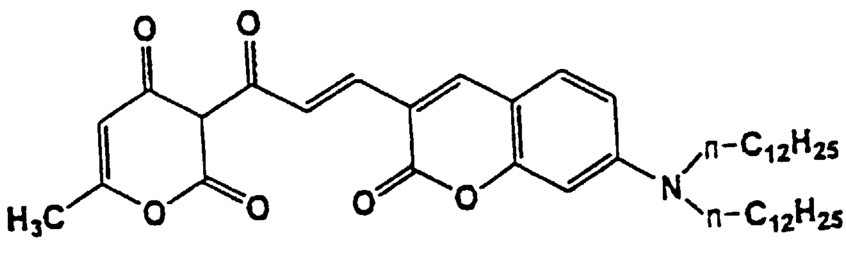

Formula 53:

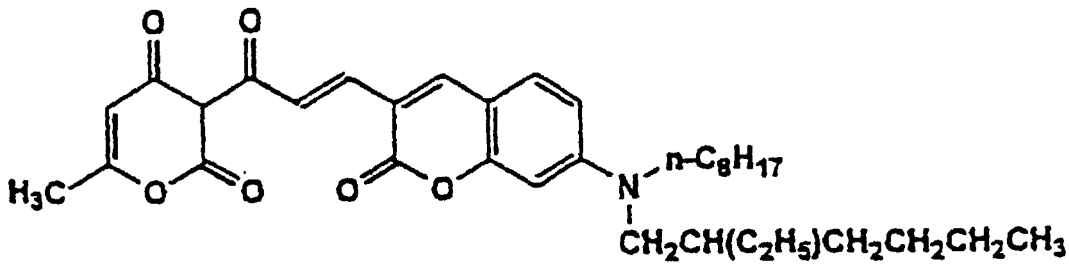

Formula 54:

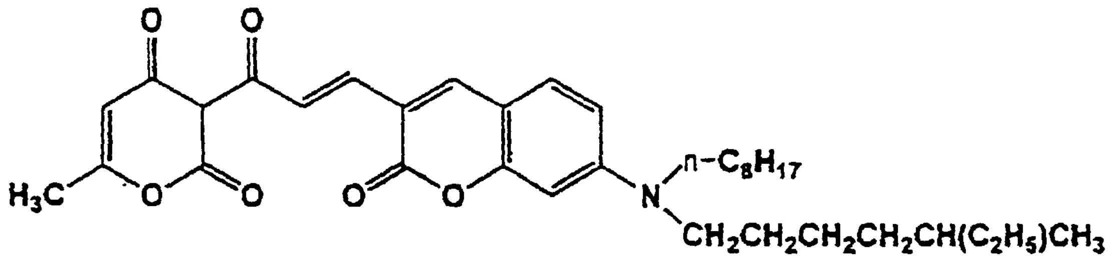

Formula 55:

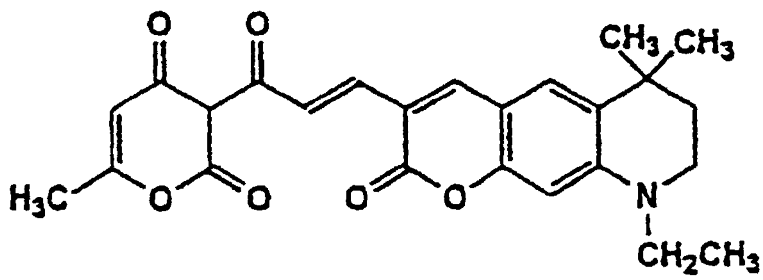

Formula 56:

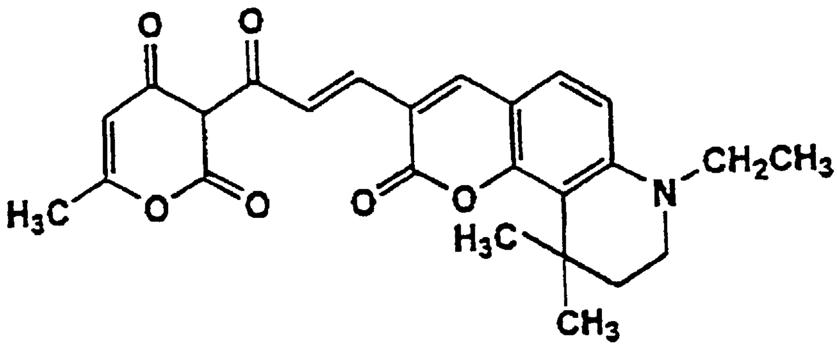

Formula 57:

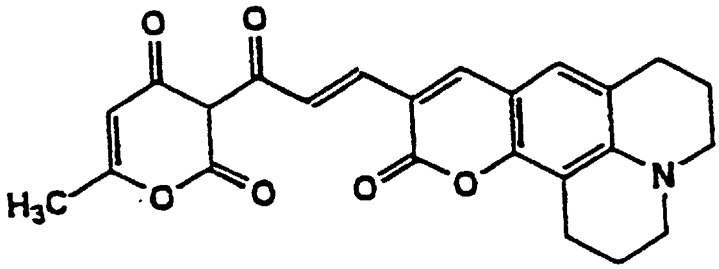

Formula 58:

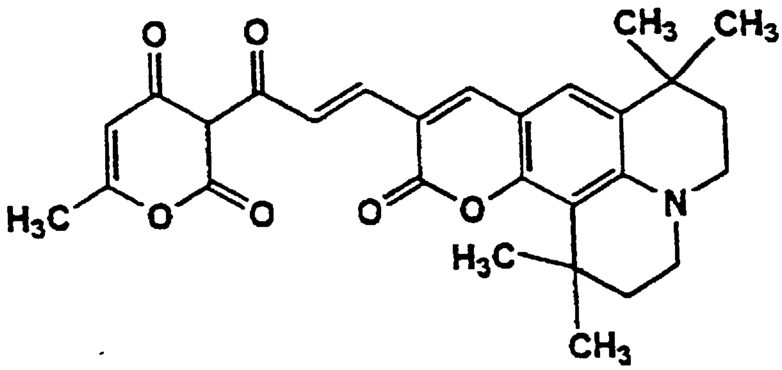

Formula 59:

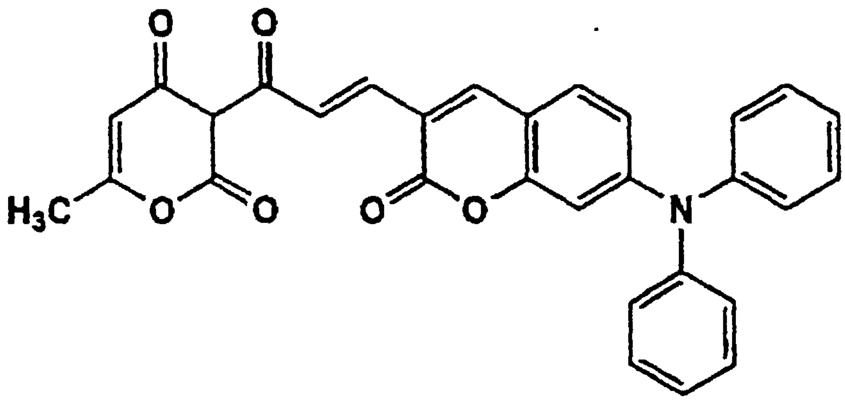

Although the pyran derivatives of the present invention can be prepared in different methods, for example, the following methods can be preferably used when economy is valued: A method to produce the pyran derivatives through a step of either reacting dehydroacetic acid with 3-formylcoumarin, represented by Formula 60 which has hydrogen atom or a desired substituent for $R_1$, $R_2$ and $R_4$ as in Formula 1 to 3; or reacting the 3-formylcoumarin with 4-cyanomethylene-2,6-dimethyl-4H-pyran, represented by Formula 61 having the desired substituent $R_3$ as in Formula 1. According to the method as disclosed in Japanese Patent Kokai No. 95,243/96, the 3-formylcoumarin represented by Formula 60 is first prepared, and then allowed to react with dehydroacetic acid to obtain the pyran derivative represented by Formula 3. To prepare the pyran derivative represented by Formula 2, the pyran derivative represented by Formula 3 is allowed to react, for example, with an acid such as hydrochloric acid, hydrofluoric acid, sulfuric acid, nitric acid, phosphoric acid, or perchloric acid. To prepare the pyran derivative represented by Formula 1, the pyran derivative represented by Formula 2 is allowed to react, for example, with the active cyanomethylene compound represented by Formula 62 where $R_3$ is hydrogen atom or a desired substituent; or the compound with 3-formylcoumarin skeleton, represented by Formula 60, is allowed to react with the compound with 4-cyanomethylene-2,6-dimethyl-4H-pyran skeleton, represented by Formula 61.

Formula 60:

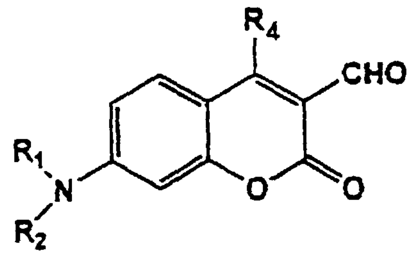

Formula 61:

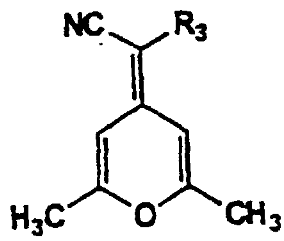

$NC-CH_2-R_3$ Formula 62

The pyran derivatives thus obtained may be used intact depending on use, however, they are usually purified prior to use by conventional methods used generally in the related compounds, for example, separation, decantation, filtration, concentration, thin-layer chromatography, column chromatography, gas chromatography, high-performance liquid chromatography, distillation, crystallization, and sublimation; and these methods can be used in combination, if necessary. In the case of using as photosensitizers, the pyran derivatives should preferably be at least distilled and/or crystallized, while in the case of using in organic EL devices, the derivatives should preferably be further purified by a method such as sublimation to suit to their final use.

As described above, since the pyran derivatives of the present invention have distinct sensitivity to lights with longer wavelengths, particularly, visible light, and have features that they effectively sensitize different polymerizable compounds and polymerization initiators; they can be extremely useful as photosensitizers to polymerize photochemically the polymerizable compounds by using lights with longer wavelengths than ultraviolet ray, particularly, by using visible light. In use, the pyran derivatives of the present invention can be usually prepared into compositions such as polymerizable compounds, polymerization initiators, and binding resins. However, depending on the type of the pyran derivatives and the final use of photopolymerizable compounds, polymerization initiators and/or binding resins may be omitted.

The polymerizable compounds, which the photosensitizers of the present invention are applicable to, are, for example, monomers, oligomers, prepolymers, and mixtures thereof, which contain at least one polymerizable multiple bond such as ethylenic-double-bond intramolecularly. Examples of such polymerizable compounds are ethylacrylate, hydroxyethylacrylate, ethyleneglycoldimethacrylate, pentaerythritol triacrylate, pentaerythritol trimethacrylate, polyester methacrylate, polyurethane methacrylate, and epoxymethacrylate. However, the polymerizable compounds, which are incorporated into the photopolymerizable compositions of the present invention, should not be restricted to the above compounds and include all polymerizable compounds which can be photochemically polymerized by using the photosensitizers of the present invention.

Examples of the polymerization initiators include any organic peroxides, 2,4,6-trichloromethyl-s-triazine, benzoylalkylether, bisimidazol, iron-allene complexes, titanocene compounds, N-phenylglycine, and diphenyliodonium salts, which are conventional polymerization initiators used in photopolymerization and which can be used in combination, if necessary. The organic peroxides are, for example, peroxyesters including di-t-butyldiperoxyisophthalate and 3,3'-4,4'-tetrakis(t-butyldioxycarbonyl)benzophenone; dialkyl peroxides including 2,5-dimethyl-2,5-bis(t-butyldioxy)-3-hexane and di-t-butylperoxide; hydroperoxides including 2,5-bis(hydroperoxy)-2,5-dimethylhexane and t-butylhydroperoxide; peroxy acetals including butyl-4,4-bis(t-butyldioxy)valylate and 1,1-bis(t-butyldioxy)-3,3,5-trimethylcyclohexane; and ketone peroxides including ethyl methyl ketone.

Varied depending on use, any binding resins, which are generally used in photopolymerizable compositions, can be used in the present invention. Respective examples of such binding resins are poly-N-vinylpyrrolidone, poly(vinyl acetate), poly(vinyl butyral), poly(vinyl carbazole), polystyrene, poly(methyl methacrylate), poly(ethylene oxide), poly(butyl methacrylate), styrene-maleicesther, poly(methyl methacrylate)-methacrylic acid or the like, and poly-N-vinylpyrrolidoneglycidyl methacrylate resins.

The photosensitizers of the present invention include one or more of the pyran derivatives of the present invention. Usually, photopolymerizable compositions are prepared by incorporating into one part by weight of any of the photosensitizers of the present invention 1-1,000 parts by weight, and preferably 10-500 parts by weight of any of the aforesaid polymerizable compounds; and if necessary, further incorporating into the mixtures any of the aforesaid binding resins in an amount up to 1,000 parts by weight, and preferably up to 500 parts by weight, and incorporating into the resulting mixtures any of the aforesaid polymerization initiators in an amount of 0.1-10 parts by weight, and preferably 0.5-5 parts by weight. In addition, the following quinoid or phenolic thermopolymerization inhibitors such as hydroquinone, pyrogallol, and 2,6-di-t-butyl-p-cresol; and plasticizers such as saturated- or unsaturated-carboxylic esters including phthalic acid esters adipic acid esters can be appropriately added to the above photopolymerizable compositions. The polymerization method used in the present invention is not specifically restricted and, for example, it may be photoinitiation polymerization where light participates only in the initiation stage for radical-, ion-, and ring opening-polymerizations for polymerizable compounds; or may be photo-polyaddition polymerization where light participates in the propagation stage.

The present invention also provides the uses of the pyran derivatives in organic EL devices. Since the pyran derivatives of the present invention have luminous ability and hole/electron transportation ability and function as dye agents for doping other luminescent compounds, the derivatives can be extremely-advantageously used both in the dispersion- and the evaporation-types of organic EL devices which need one or more of the above features as essential factors. The organic EL devices of the present invention intrinsically contain at least one layer which comprises an organic compound and incorporates the pyran derivative represented by Formula 1, 2 or 3. Particularly, the organic EL devices usually comprise an anode to be energized with positive voltage, hole transportation layer with hole transportation ability, luminous layer with luminous ability, electron transportation layer with electron transportation ability, and cathode to be energized with negative voltage. Depending on the properties of organic compounds used, one layer may also have functions as of two or more layers. The present invention does not exclude the following embodiment; one or more thin layers, which contain appropriate substances such as lithium fluoride and triazole derivatives, are inserted between the above anode and cathode and between the above layers each to improve the adhesion level of each adjacent layers each, hole implantation rate, electron implantation rate, and/or electric conductivity of electrodes.

Explaining now the organic EL devices of the present invention with reference to an evaporation-type organic EL device as shown in FIG. 1, the numeral 1 represents a substrate which is usually made of a substrate material in general including glasses such as soda glass, barium boron silicate glass, and aluminosilicate glass; plastics; or ceramics. Thus, when other displaying devices and circuit means such as operation circuits and regulation circuits are closely provided each other, the substrate 1 can be shared with the above devices and circuits.

The numeral 2 represents an anode which is usually formed by attaching closely to one side of the substrate 1 by vacuum deposition, etc., and then coating thereupon an electric conductive material, which has electrical low-resistivity and high-transmissivity through over the whole visible region, to form a thin layer having usually a thickness of 10-1,000 nm. As the electric conductive material, tin oxide or a mixture system of tin oxide and indium oxide (hereinafter abbreviated as "ITO") can be used. Among these, ITO can be extremely advantageously used in the present invention because it can be easily processed into low-resistive ones and etched with acids to form minute patterns easily.

The numeral 3 represents a hole transportation layer which is usually formed by attaching closely to the anode 2 by vacuum deposition, etc., and then coating thereupon pyrazoline or an aromatic tertiary amine which has as a main skeleton triphenylamine such as N,N'-bis(3-methylphenyl)-N,N'-diphenyl-(1,1'-biphenyl)-4,4'-diamine(hereinafter abbreviated as "TPD"), represented by formula 66, to form a thin layer having usually a thickness of 1-1,000 nm.

Formula 66:

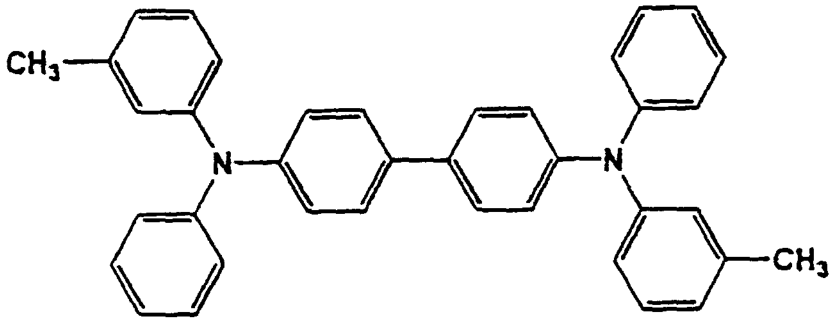

The numeral 4 represents a luminous layer which is usually formed by attaching closely to the hole transportation layer 3, and then coating thereupon by vacuum deposition, etc., compounds capable of emitting light or luminescent compounds to form a thin layer having usually a thickness of 1-1,000 nm. Examples of the compounds capable of emitting light are the pyran derivatives of the present invention, oxathiazol, and derivatives thereof, and metal complexes of aluminum, zinc, and beryllium which comprise as a ligand 8-hydroxyquinoline or derivatives thereof, which all can be appropriately used in combination, if necessary. The luminous layer 4 may be omitted when a compound, which is to be incorporated into the hole transportation layer 3 and/or the later described electron transportation layer, has also luminous ability.

The numeral 5 represents an electron transportation layer which is formed by attaching closely to the luminous layer 4 and then coating thereupon either the same compound similarly as in the luminous layer 4 or a cyclic ketone such as benzoquinone, anthraquinone, freorenon, or derivatives thereof to form a thin layer having usually a thickness of 10-500 nm.

The numeral 6 represents a cathode which is usually formed by attaching closely to the electron transportation layer 5 and then co-evaporating a metal such as lithium, magnesium or calcium having a lower work-function of, usually, not higher than 6 eV, than that of the compound used in the electron transportation layer; and a metal such as silver, aluminum or indium directed to inhibit the collosion of the above metals. When a desired light transmissivity is needed in organic EL devices, they can be made to have such a transmissivity suitable for purposes by increasing or decreasing the thickness of the cathode 6. The thickness of the cathode layer is not specifically restricted, however, it is usually set to at least 500 nm in view of electric conductivity, production cost, and thickness of the total size of the devices, etc.

Thus, the organic EL devices of the present invention can be obtained by successively placing on the substrate 1 the anode 2, the hole transportation layer 3, the luminous layer 4, the electron transportation layer 5, and the cathode 6 while closely attaching together each adjacent layer for systematic formulation. As described above, since the pyran derivatives of the present invention also function as substances having luminous ability, hole transportation ability, or electron transportation ability, and as dye agents, i.e., dopants for doping other luminescent compounds, the derivatives can be advantageously used alone or in combination with appropriate other compounds in any of hole transportation layer, luminous layer, and electron transportation layer. It may be varied depending on final use, in the organic EL device as shown in FIG. 1, the incorporation of the pyran derivative of the present invention into the luminous layer 4 to form a luminous layer, which also acts as hole- and/or electron-transportation-layers, can omit the hole transportation layer 3 and/or the electron transportation layer 5 which use other compounds.

In the case of the organic EL device in FIG. 1, when a dc voltage is energized between the anode 2 and the cathode 6, holes injected from the anode 2 move to the luminous layer 4 through the hole transportation layer 3, while electrons injected from the cathode 6 move to the luminous layer 4 through the electron transportation layer 5. As a result, the holes and electrons couple again in the luminous layer 4, and then the formed luminescent compounds in an exited state emit a prescribed wavelength of light through the anode 2 and the substrate 1.

The preferred embodiments according to the present invention are described hereinafter with reference to the following Examples:

Example 1

Pyran derivative

2.86 g of dehydroacetic acid and 3.97 g of 7-diethylamino-3-formylcoumarin, obtained by a conventional method, were dissolved in 20 ml chloroform. The solution was admixed with 0.3 ml piperidine, and the mixture was refluxed for five hours by heating, followed by cooling the reaction mixture to ambient temperature and filtering the mixture to collect the formed crystals. The crystals were recrystallized with N,N-dimethylformamide to obtain 3.1 g of a red crystal of the pyran derivative, represented by Formula 49.
The pyran derivative had a melting point of 232-235°C and exhibited an absorption- and fluorescent-maxima at wavelengths of 493 nm and 605 nm, respectively, when measured in methylene chloride.

Example 2

Pyran derivative

4.4 g of a pyran derivative, obtained by the method in Example 1, were provided and reacted by heating at 120°C for four hours in 15 ml concentrated hydrochloric acid. The reaction mixture was cooled to ambient temperature, diluted with water, and neutralized by the addition of a 25% aqueous sodium hydroxide solution. The formed crystals were collected by filtering, refluxed by heating in methanol, and cooled to ambient temperature, followed by collecting the formed crystals by filtering to obtain 3.3 g of an orange crystal of the pyran derivative, represented by Formula 35.
The pyran derivative had a melting point of 211-213°C and exhibited an absorption- and fluorescent-maxima at wavelengths of 454 nm and 513 nm, respectively, when measured in methylene chloride.

Example 3

Pyran derivative

Three grams of a pyran derivative, obtained by the method in Example 2, were provided and reacted with 0.68 g malononitrile in 12 ml acetic anhydride by heating at 140°C for four hours. Thereafter, the reaction mixture was cooled to ambient temperature and filtered to collect the formed crystals. The crystals were recrystallized with N,N-dimethylformamide to obtain two grams of a brilliant red-violet crystal of the pyran derivative, represented by Formula 5.
The pyran derivative had a melting point of 264-266°C and exhibited an absorption- and fluorescent-maxima at wavelengths of 488 nm and 590 nm, respectively, when measured in methylene chloride.

Example 4

Pyran derivative

Three and half grams of a pyran derivative, obtained by the method in Example 2, were provided and reacted with 2.3 g ethyl cyanoacetate in 42 ml acetic anhydride by heating at 140°C for four hours. Thereafter, the reaction mixture was cooled to ambient temperature and filtered to obtain the formed crystals. The crystals were recrystallized with N,N-dimethylformamide to obtain 1.1 g of a red-violet crystal of the pyran derivative, represented by Formula 16.
The pyran derivative had a melting point of 200-203°C and exhibited an absorption- and fluorescent-maxima at wavelengths of 479 nm and 588 nm, respectively, when measured in methylene chloride.

Example 5

Pyran derivative

One gram of a pyran derivative represented by Formula 37, obtained in accordance with the method in Example 2, was provided and reacted with 0.5 g of 2-cyanomethylbenzothiazole in 8 ml of acetic anhydride by heating at 140°C for four hours. The reaction mixture was cooled to ambient temperature and filtered to obtain the formed crystals. The crystals were recrystallized in a mixture of chloroform and methanol to obtain 0.8 g of a red-violet crystal of the pyran derivative, represented by Formula 20.
The pyran derivative had a melting point of 224-226°C and exhibited an absorption maximum at a wavelength of 516 nm when measured in methylene chloride.

Example 6

Pyran derivative

A compound represented by Formula 63, obtained by a conventional method, and 7-dibutylamino-3-formylcoumarin in respective amounts of 2.2 g and 1.5 g were provided and dissolved by heating in 15 ml N,N-dimethylformamide, further admixed with 0.4 ml piperidine, and reacted at 100°C for one hour. The reaction mixture was cooled to ambient temperature, admixed with 15 ml methanol, and filtered to collect the formed crystals. The crystals were recrystallized with a mixture of chloroform and methanol to obtain 0.9 g of a red-violet crystal of the pyran derivative, represented by Formula 20.

Formula 63:

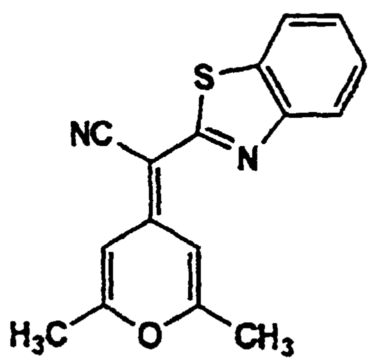

The pyran derivative had the same melting point as of the compound in Example 5.

Example 7

Pyran derivative

1.1 g of a pyran derivative, obtained by the method in Example 2, was provided and reacted with 0.57 g 2-cyanomethylbenzoxazole in 12 ml acetic anhydride by heating at 140°C for four hours. The reaction mixture was cooled to ambient temperature and filtered to collect the formed crystals. The crystals were recrystallized with N,N-dimethylformamide to obtain 0.84 g of a red-violet crystal of the pyran derivative, represented by Formula 25.

The pyran derivative had a melting point of 262-264°C and exhibited an absorption maximum at a wavelength of 510 nm when measured in methylene chloride.

Example 8

Pyran derivative

Three and half grams of a pyran derivative, obtained by the method in Example 2, was reacted with 1.25 g cyanoacetoamide in 42 ml acetic anhydride by heating at 140°C for four hours. The reaction mixture was cooled to ambient temperature and filtered to collect the formed crystals. The crystals were purified on silica gel chromatography to obtain 0.6 g of a brilliant red-violet crystal of the pyran derivative, represented by Formula 17.

The pyran derivative had a melting point of 263-265°C and exhibited an absorption- and fluorescent-maxima at wavelengths of 487 nm and 590 nm, respectively, when measured in methylene chloride.

Example 9

Pyran derivative

Compounds represented by Formulae 63 and 64, obtained by a conventional method, were respectively weighted in amounts of 1.8 g and 2.0 g and dissolved by heating in 15 ml N,N-dimethylformamide, admixed with 0.3 ml

piperidine, and reacted at 100°C for one hour. Thereafter, the reaction mixture was cooled to ambient temperature, admixed with 15 ml methanol, and filtered to collect the formed crystal. The crystal was dissolved by heating in an adequate amount of N,N-dimethylformamide, filtered, and admixed with 15 ml methanol, followed by collecting the formed crystal to obtain 0.8 g of a red-purple crystal of the pyran derivative, represented by Formula 23.

Formula 64:

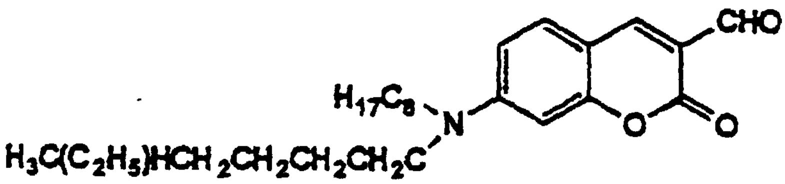

The pyran derivative had a melting point of 160-164°C and exhibited an absorption maximum at a wavelength of 519 nm when measured in methylene chloride.

Example 10

8.20 g of a compound represented by Formula 64, obtained by a conventional method, and 3.36 g dehydroacetic acid were dissolved in 30 ml chloroform, admixed with 0.5 ml piperidine, and refluxed by heating for five hours. The reaction mixture was cooled to ambient temperature, admixed with 60 ml methanol, and filtered to collect the formed crystal. The crystal was recrystallized with a mixture solution of chloroform and methanol to obtain 2.8 g of a red crystal of the pyran derivative, represented by Formula 54.

The pyran derivative had a melting point of 160-162°C and exhibited an absorption- and fluorescent-maxima at wavelengths of 498 nm and 605 nm, respectively, when measured in methylene chloride.

Example 11

Pyran derivative

Six grams of a pyran derivative, obtained by the method in Example 10, was provided, admixed with acetic acid and concentrated hydrochloride in respective amounts of 60 ml and 30 ml, and reacted by heating at 120°C for seven hours. The reaction mixture was cooled to ambient temperature, diluted with water, and neutralized by the addition of a 25% aqueous sodium hydroxide solution, followed by filtering the mixture to collect the formed crystal. By applying the crystal to silica gel column chromatography using a mixture solution of chloroform and methanol as an eluent, 4.2 of an orange crystal of the pyran derivative, represented by Formula 41, was obtained.

The pyran derivative had a melting point of 84-87°C and exhibited an absorption- and fluorescent-maxima at wavelengths of 460 nm and 511 nm, respectively, when measured in methylene chloride.

Example 12

Pyran derivative

Three grams of a pyran derivative, obtained by the method in Example 11, was provided, admixed with acetic anhydride, and malononitrile in respective amounts of 12 ml and 0.42 g, and reacted by heating at 120°C for four hours. The reaction mixture was cooled to ambient temperature, and the formed crystal was collected by filtering and recrystallized with a mixture solution of N,N-dimethylformamide and methanol to obtain 1.9 g of a brown crystal of the pyran derivative, represented by Formula 10.

The pyran derivative had a melting point of 176-177°C and exhibited an absorption- and fluorescent-maxima at wavelengths of 491 nm and 593 nm, respectively, when measured in methylene chloride.

The properties of preferred examples of the compounds according to the present invention, which include the pyran derivatives in the above Examples, are tabulated in Table 1. In Table 1, the wavelengths of absorption maxima of these compounds were measured after dissolved in methylene chloride, and their fluorescent spectra were measured in methylene chloride at a concentration of $10^{-7}$M. As a control, a conventional pyran derivative, represented by

Formula 65, was used.

Formula 65:

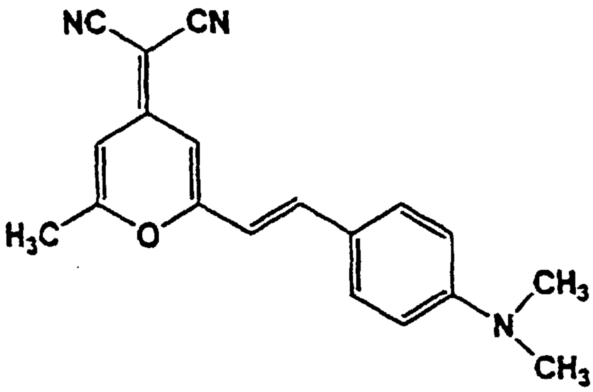

Table 1

| Compound | Absorption maximum (nm) | Fluorescence | | Melting point (°C) |
|---|---|---|---|---|
| | | Maximum wavelength (nm) | Intensity | |
| Formula 5 | 488 | 590 | 4.51 | 264-266 |
| Formula 6 | 490 | 592 | 4.82 | 256-258 |
| Formula 8 | 491 | 592 | 5.08 | 189-190 |
| Formula 10 | 491 | 593 | 5.15 | 176-177 |
| Formula 16 | 479 | 588 | 1.91 | 200-203 |
| Formula 17 | 487 | 590 | 4.57 | 263-265 |
| Formula 19 | 515 | - | - | 253-255 |
| Formula 20 | 516 | - | - | 224-226 |
| Formula 21 | 517 | - | - | 140-142 |
| Formula 23 | 519 | - | - | 160-164 |
| Formula 25 | 510 | - | - | 262-264 |
| Formula 26 | 508 | - | - | 279-282 |
| Formula 35 | 454 | 513 | 7.27 | 211-213 |
| Formula 37 | 459 | 504 | 8.73 | 167-169 |
| Formula 39 | 459 | 504 | 8.38 | 113-115 |
| Formula 41 | 460 | 511 | 8.86 | 84-87 |
| Formula 49 | 493 | 605 | 5.69 | 232-235 |
| Formula 50 | 497 | 604 | 4.78 | 236-237 |
| Formula 52 | 500 | 605 | 6.22 | 173-174 |
| Formula 54 | 498 | 605 | 6.29 | 160-162 |
| Formula 65 (Control) | 468 | 589 | 1.79 | 222-224 |

As evident from the results in Table 1, the absorption maxima of the pyran derivatives of the present invention clearly shifted to the side of a longer wavelength as compared with the control. Particularly, the wavelengths of absorption maxima of the pyran derivatives, represented by Formulae 5, 6, 8, 10, 16, 17, 49, 50, 52 and 54, are close to the wavelength of 488 nm by argon laser; and those of the pyran derivatives, represented by Formulae 19, 21, 23, 25 and 26, are close to the second harmonic wavelength of 532 nm by YAG laser. This demonstrates that the pyran derivatives of the present invention are useful as photosensitizers which polymerize polymerizable compounds with visible light. As far as the fluorescent measurement in this example, no fluorescence was observed in the pyran derivatives represented by Formulae 19, 21, 23, 25 and 26, however, the other pyran derivatives showed at least two-fold stronger luminous ability than that of control. The data indicates that the pyran derivatives of the present invention have a relatively-high field luminous ability and laser action.

Example 13

Photopolymerizable composition

According to the usual manner, 4-types of photopolymerizable compositions were prepared by mixing 900 parts by weight of ethyl cellosolve with 100 parts by weight of pentaerythritol acrylatemonomer as a photopolymerizable compound, 100 parts by weight of acrylic acid/methacrylic acid copolymer as a binder, eight parts by weight of 3,3',4,4'-tetra(t-butylperoxycarbonyl)benzophenone as a polymerization initiator, and two parts by weight of the pyran derivative, represented by Formula 10, 23, 41 or 54, as a photosensitizer.

The above photopolymerizable compositions were homogeneously coated over sand-set aluminum plates, which the surfaces had been treated, to form photosensitive layers, followed by forming polyvinyl alcohol layers on the formed photosensitive layers to protect the polymerization inhibition by oxygen. A grace scale was closely attached to each of the photosensitive layers and, after installing a 150 W xenon lump, was irradiated by a light of 488 nm, corresponding to the light of argon laser, and a light of 532 nm, corresponding to the light of second harmonic by YAG laser, which were both obtained by combining sharp cult filters ("Y47" and "Y52") commercialized by Toshiba Glass Kabushiki Kaisha, Tokyo, Japan; interference filters ("KL49" and "KL54") commercialized by Toshiba Glass Kabushiki Kaisha, Tokyo, Japan; and a heat-ray-cut-filter ("HA30") commercialized by HOYA Corporation, Tokyo, Japan. Thereafter, the irradiated layers were in the usual manner developed in an alkaline developer, followed by calculating the sensitivity based on the step number at which the compositions were photo-cured in such a manner that, in the numerical formula represented by the following equation, transmittance Tn of the step number "n" of a step tablet, exposure time t, and exposure strength Io were respectively substituted for the equation. In parallel, in place of the pyran derivatives of the present invention, a control system using a conventional compound, represented by Formula 65, was provided and treated similarly as above. The results are in Table 2.

$$E(mJ/cm^2) = Io(mJ/cm^2 \bullet s) \times Tn \times t(s) \qquad \text{Equation}$$

Table 2

| Compound | Wavelength, measured | Sensitivity ($mJ/cm^2$) |
|---|---|---|
| Formula 10 | 488 nm | 0.4 |
| Formula 23 | 488 nm | 2.0 |
| Formula 41 | 488 nm | 0.6 |
| Formula 54 | 488 nm | 10 |
| Formula 65 (Control) | 488 nm | 1.4 |
| Formula 10 | 532 nm | 1.3 |
| Formula 23 | 532 nm | 1.5 |
| Formula 41 | 532 nm | 12 |
| Formula 54 | 532 nm | 16 |
| Formula 65 (Control) | 532 nm | 2.8 |

As evident from the results in Table 2, the pyran derivatives represented by Formulae 10 and 41 of the

present invention showed a sensitivity as high as about two-fold of that of the conventional pyran derivative represented by Formula 65. When measured at a wavelength of 532 nm, the pyran derivatives represented by Formulae 10 and 23 of the present invention exhibited a sensitivity as high as about two-fold of that of the control. The data demonstrates that the pyran derivatives of the present invention are useful to sensitize polymerizable compounds and polymerization initiators in photochemical polymerization.

Example 14

Organic EL device

A glass substrate having a transparent ITO electrode, 100 nm in thickness, which had been patternized by a vaporized aqua regia, was ultrasonically washed with a neutral detergent, refined water, and isopropyl alcohol, and then taken out from the boiling isopropyl alcohol, dried, washed with ultraviolet ozone, and fixed on a vaporizing apparatus, followed by lowering the inner pressure to $10^{-6}$ Torr. Thereafter, a hole transportation layer was formed by vaporizing TPD on the surface of the glass substrate, having the transparent ITO electrode as an anode, to form a TPD layer, 50 nm in thickness, at a vaporization rate of 1 nm/sec. On the hole transportation layer were co-evaporated both the pyran derivative, represented by Formula 49 obtained by the method in Example 1, and tris(8-hydroxyquinoline)aluminum (hereinafter abbreviated as "Alq3") in a weight ratio of 1.8:100 up to form a layer, 15 nm in thickness, at an evaporation rate of 0.5 nm/sec and to obtain a luminous layer; and further evaporated with Alq3 to form a layer, 45 nm in thickness, at a evaporation rate of 1 nm/sec to form an electron transportation layer, followed by co-evaporating magnesium and silver (=10:1 by weight) in combination at an evaporation ratio of 1 nm/sec up to form a layer as a cathode, 200 nm in thickness.

When energized with a dc current voltage of 14 V, the organic EL device thus obtained emitted a red luminescence having a brightness of 6,200 cd/$m^2$ and a luminescent maximum wavelength of 626 nm at a current density of 400 mA/$cm^2$. The luminescence stably lasted under dry nitrogen atmospheric conditions and no partial dark spot was observed even at 5,000 hours after the initiation of the emission. The half life of the luminescent brightness, having an initiation brightness of 200 cd/$m^2$, was about 3,000 hours when operated at a constant current of 10 mA/$cm^2$.

Example 15

Organic EL device

An organic EL device was prepared similarly as in Example 14 except that the step for preparing electron transportation layer was omitted and that, when forming a luminous layer, Alq3 and the pyran derivative, represented by Formula 49 obtained by the method in Example 14, were co-evaporated in the same weight ratio as used in Example 14 up to form a luminous layer, 60 nm in thickness, which also acted as an electron transportation layer.

The organic EL device obtained in this Example gave a red luminescence which had a brightness of 6,500 cd/$m^2$ and a luminescent maximum wavelength of 625 nm at a current density of 420 mA/$cm^2$ when energized with a dc current voltage of 14 V.

Example 16

Organic EL device

An organic EL device was prepared similarly as in Example 14 except that the step for preparing an electron transportation layer was omitted and the use of Alq3 was also omitted when forming a luminous layer, and that the pyran derivative, represented by Formula 49 obtained by the method in Example 1, was evaporated up to form a luminous layer, 50 nm in thickness, which also acted as an electron transportation layer.

The organic EL device obtained in this Example gave a red luminescence having a brightness of 4,000 cd/$m^2$ and a luminescent maximum wavelength of 630 nm at a current density of 380 mA/$cm^2$ when energized with a dc current voltage of 18 V.

Example 17

Organic EL device

An organic EL device was prepared similarly as in Example 14 except that, when forming a luminous layer, the pyran derivative represented by Formula 49 was replaced with the pyran derivative represented by Formula 35,

obtained by the method in Example 2, and then the substituted pyran derivative and Alq3 were co-evaporated in a weight ratio of 2.5:100 up to form a layer, 15 nm in thickness.

The organic EL device obtained in this Example gave a luminescence having a brightness of 900 cd/$m^2$ and a luminescent maximum wavelength of 535 nm at a current density of 520 mA/$cm^2$ when energized with a dc current voltage of 14 V.

Example 18

Organic EL device

An organic EL device was prepared similarly as in Example 14 except that the step for preparing an electron transportation layer was omitted and, when in the case of forming a luminous layer, the pyran derivative represented by Formula 49 was replaced with the pyran derivative represented by Formula 35 obtained by the method in Example 2, and then Alq3 and the substituted pyran derivative were co-evaporated in a weight ratio of 2.5:100 up to form a luminous layer, 60 nm in thickness, which also acted as an electron transportation layer.

The organic EL device obtained in this Example gave a luminescence having a brightness of 4,500 cd/$m^2$ and a luminescent maximum wavelength of 535 nm at a current density of 460 mA/$cm^2$ when energized with a dc current voltage of 14 V.

Example 19

Organic EL device

An organic EL device was prepared similarly as in Example 14 except that, when in the case of forming a luminous layer, the pyran derivative represented by Formula 49 was replaced with the pyran derivative represented by Formula 5, obtained by the method in Example 3, and then the substituted pyran derivative and Alq3 were co-evaporated in a weight ratio of 2.5:100 up to form a luminous layer, 15 nm in thickness.

The organic EL device obtained in this Example gave a luminescence having a brightness of 6,200 cd/$m^2$ and a luminescent maximum wavelength of 610 nm at a current density of 520 mA/$cm^2$ when energized with a dc current voltage of 14 V.

Example 20

Organic EL device

An organic EL device was prepared similarly as in Example 14 except that the step for forming an electron transportation layer was omitted and that, when forming a luminous layer, the pyran derivative represented by Formula 49 was replaced with the pyran derivative represented by Formula 5, obtained by the method in Example 3, followed by co-evaporating the substituted pyran derivative and Alq3 in a weight ratio of 2.5:100 up to form a luminous layer, 60 nm in thickness, which also acted as an electron transportation layer.

The organic EL device obtained in this Example gave a luminescence having a brightness of 5,800 cd/$m^2$ and a luminescent maximum wavelength of 610 nm at a current density of 420 mA/$cm^2$ when energized with a dc current voltage of 15 V.

Example 21

Organic EL device

An organic EL device was prepared similarly as in Example 14 except that, when forming a luminous layer, Alq3 was omitted and that the pyran derivative represented by Formula 49 was replaced with the pyran derivative represented by Formula 5, obtained by the method in Example 3, followed by evaporating only the replaced pyran derivative up to form a luminous layer, 50 nm in thickness.

The organic EL device obtained in this Example gave a luminescence having a brightness of 3,200 cd/$m^2$ and a luminescent maximum wavelength of 625 nm at a current density of 380 mA/$cm^2$ when energized with a dc current voltage of 18 V.

INDUSTRIAL APPLICABILITY

As described above, the present invention was made based on the creation of novel pyran derivatives and the finding of their industrial useful properties. Since the pyran derivatives have a distinctive sensitivity to visible light, they can be extremely useful as photosensitizers for photochemically polymerizing photopolymerizable compounds by irradiating visible light such as argon laser, helium/neon laser, the second harmonic by YAG laser, etc., in the presence or absence of a polymerization initiator. Accordingly, the pyran derivatives of the present invention can be extremely useful in a variety of fields including information recordings and electric instruments and apparatuses such as PS plates for printings and plate makings, paints, photo-recovering adhesives, insulating materials for printing substrates, digital color proofs, and holographies, which all inevitably need photopolymerization using the above lasers as luminous sources.

Because the pyran derivatives of the present invention have a distinctive luminous ability, they can be extremely useful as dyes for doping both luminescent compounds in organic EL devices and other luminescent compounds, and useful as laser-active substances in dye lasers.

## Claims

1. A pyran derivative represented by any one of the following Formulae 1 to 3:

Formula 1:

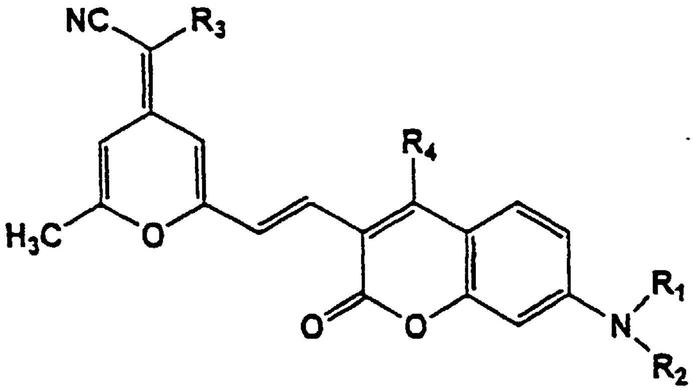

Formula 2:

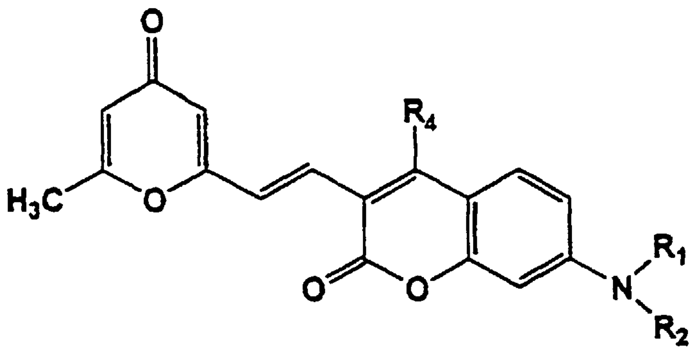

Formula 3:

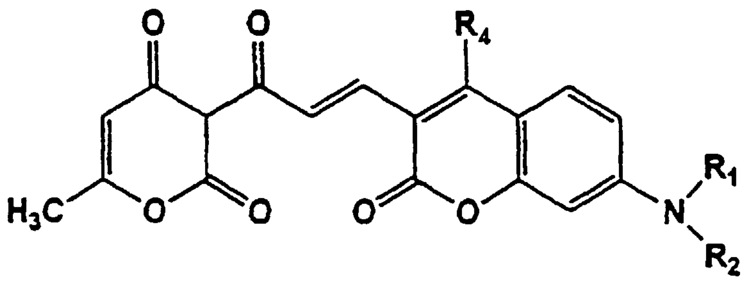

where $R_1$ and $R_2$ are independently hydrogen or a straight- or branched-chain alkyl group, alkenyl group, or aryl group, wherein the alkyl and aryl groups may have a substituent; $R_3$ is cyano group, carboxylic acid group, carboxylic acid ester group, amido group, or a five- or six-membered heterocycle which may form a fused ring; $R_4$ is hydrogen, halogeno, cyano, carbonyl group having up to five carbon atoms, or a substituent derivable from the carbonyl group provided that $R_1$ and/or $R_2$ may form a ring structure along with the nitrogen to which they bound together with or without benzene ring bonded to the nitrogen.

**2.** A photosensitizer which comprises the pyran derivative of claim 1.

**3.** A photopolymerizable composition which comprises the pyran derivative of claim 1.

**4.** The photopolymerizable composition of claim 3, which comprises a polymerization initiator and/or a binding resin in combination with a polymerizable compound.

5. The photopolymerizable composition of claim 3 or 4, which can be polymerized by irradiating visible light.

6. An organic electroluminescent device comprising at least one layer made of a layer which comprises an organic compound and into which the pyran derivative of claim 1 is incorporated.

7. The organic electroluminescent device of claim 6, which emits visible light when energized with a dc voltage.

8. The organic electroluminescent device of claim 6 or 7, which comprises an anode layer, a layer having a hole transportation ability, a layer capable of emitting light, a layer capable of transporting electron, and a cathode layer.

9. The organic electroluminescent device of claim 6, 7 or 8, wherein said at least one layer has at least two functional abilities selected from the group consisting of luminous ability, electron transportation ability, and electron transportation ability.

10. A displaying device, which uses any one of the organic electroluminescent device of claim 6, 7, 8 or 9.

11. A laser oscillating device, which uses the pyran derivative of claim 1 as a laser active substance.

12. A process for producing the pyran derivative of claim 1, which comprises a step of reacting dehydroacetic acid with a compound having 3-formylcoumarin skeleton.

13. A process for producing the pyran derivative of claim 1, which comprises a step of reacting a compound having 3-formylcoumarin skeleton with a compound having 4-cyanomethylene-2, 6-dimethyl-4H-pyran skeleton.

**FIG. 1**

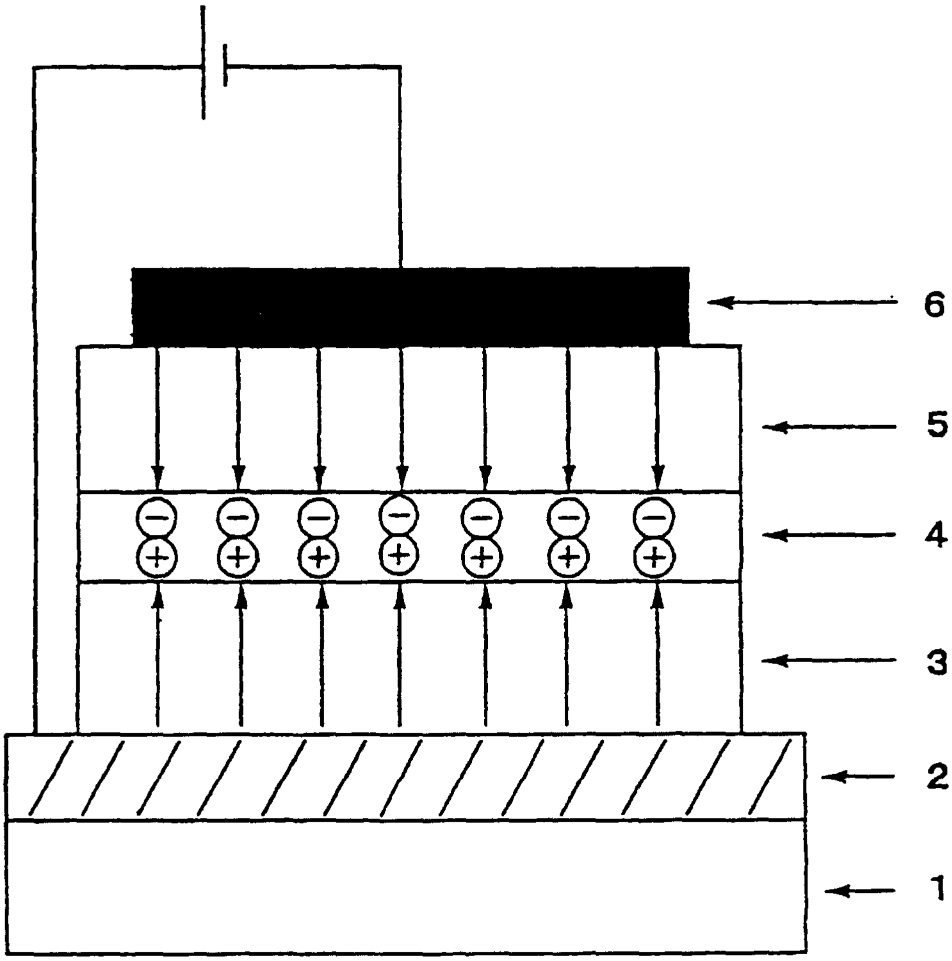

**INTERNATIONAL SEARCH REPORT**

International application No.
PCT/JP99/00459

A. CLASSIFICATION OF SUBJECT MATTER
Int.Cl[6] C07D405/14, 407/06, 413/14, 417/14, 491/052, 491/16, C08F2/50, C08K5/18, G03F7/031, H01S3/16

According to International Patent Classification (IPC) or to both national classification and IPC

B. FIELDS SEARCHED

Minimum documentation searched (classification system followed by classification symbols)
Int.Cl[6] C07D405/14, 407/06, 413/14, 417/14, 491/052, 491/16, C08F2/50, C08K5/18, G03F7/031, H01S3/16

Documentation searched other than minimum documentation to the extent that such documents are included in the fields searched

Electronic data base consulted during the international search (name of data base and, where practicable, search terms used)
CA (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| X, P | JP, 10-45741, A (Mitsui Toatsu Chemicals,Inc.), 17 February, 1998 (17. 02. 98) (Family: none) | 1-13 |
| X, P | JP, 10-195118, A (Mitsubishi Chemical Corp.), 28 July, 1998 (28. 07. 98) (Family: none) | 1-13 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 May, 1999 (20. 05. 99) | 1 June, 1999 (01. 06. 99) |
| Name and mailing address of the ISA/<br>Japanese Patent Office | Authorized officer |
| Facsimile No. | Telephone No. |

Form PCT/ISA/210 (second sheet) (July 1992)